(19) Europäisches Patentamt
European Patent Office
Office européen des brevets

(11) EP 3 957 365 A1

(12) **EUROPEAN PATENT APPLICATION**
published in accordance with Art. 153(4) EPC

(43) Date of publication:
**23.02.2022 Bulletin 2022/08**

(21) Application number: **20790589.4**

(22) Date of filing: **16.04.2020**

(51) International Patent Classification (IPC):
***A61P 27/02*** *(2006.01)* ***A61P 27/10*** *(2006.01)*
***C07D 339/04*** *(2006.01)* ***A61K 9/06*** *(2006.01)*
***A61K 9/08*** *(2006.01)* ***A61K 31/385*** *(2006.01)*
***A61K 31/496*** *(2006.01)* ***A61K 31/5377*** *(2006.01)*

(52) Cooperative Patent Classification (CPC):
**A61K 9/06; A61K 9/08; A61K 31/385; A61K 31/496; A61K 31/5377; A61P 27/02; A61P 27/10; C07D 339/04**

(86) International application number:
**PCT/JP2020/016774**

(87) International publication number:
**WO 2020/213693 (22.10.2020 Gazette 2020/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.04.2019 JP 2019078798**

(71) Applicant: **Santen Pharmaceutical Co., Ltd.**
**Kita-ku**
**Osaka-shi**
**Osaka 530-8552 (JP)**

(72) Inventors:
- **KATO, Masatomo**
  **Ikoma-shi, Nara 630-0101 (JP)**
- **ODA, Tomoko**
  **Ikoma-shi, Nara 630-0101 (JP)**
- **TAKAOKA, Shinji**
  **Osaka-shi, Osaka 530-8552 (JP)**
- **HONDA, Takahiro**
  **Ikoma-shi, Nara 630-0101 (JP)**
- **HATA, Tatsuya**
  **Ikoma-shi, Nara 630-0101 (JP)**

(74) Representative: **Müller-Boré & Partner Patentanwälte PartG mbB**
**Friedenheimer Brücke 21**
**80639 München (DE)**

(54) **LIPOIC ACID PRODRUG**

(57) The present disclosure provides and an agent for treating or preventing an eye disease such as presbyopia, comprising, as an active ingredient, a lipoic acid prodrug having a specified structure.

EP 3 957 365 A1

## Description

### Technical Field

The present invention relates to a lipoic acid prodrug, and an agent for treating or preventing an eye disease such as presbyopia, comprising a lipoic acid prodrug as an active ingredient.

### Background Art

Presbyopia is one of aging phenomena of the eye that begins around the age of 40 and is commonly called aged eyes. According to Non-Patent Document 1, presbyopia is defined as a disease state in which the accommodative amplitude decreases with aging (Age-Related Loss of Accommodation). In order to focus on something near or far away, it is necessary for the light that enters the eye to be refracted appropriately as it passes through the lens. Therefore, the eye has the function of adjusting the thickness of the lens such as contraction of the ciliary muscle located near the lens. The ocular tissues involved in the accommodation include lens, Zinn's zonule, lens capsule, and ciliary muscle. However, if the function of the ciliary muscle deteriorates due to aging, or if the lens elasticity (or, viscoelasticity) decreases, that is, the lens hardens, it becomes difficult to adjust the thickness of the lens, and it becomes difficult to focus on objects. This condition is presbyopia.

Patent Document 1 discloses experimental results in which lipoic acid can improve the elasticity of the mouse lens and may treat presbyopia, and also discloses synthetic examples of some lipoic acid derivatives (prodrugs). Lipoic acid choline ester (alias, EV06, UNR 844), one of the disclosed prodrugs, is under clinical development as an eye drop in the United States. Lipoic acid choline ester is metabolized to lipoic acid in vivo, which acts as an active metabolite. However, the condition of patients with presbyopia is diverse, and an increase in the types of therapeutic agents for eye diseases is still strongly desired so that therapeutic agents can be selected accordingly.

### Prior Art Document

#### Patent Document

Patent Document 1: WO 2010/147957

#### Non-Patent Document

Non-Patent Document 1: "Atarashii ganka" [A New Ophthalmology], Vol. 28, No.7, 985-988, 2011

The disclosures of the prior art documents cited herein are hereby incorporated by reference in their entirety.

### Summary

#### Technical Problem

An object of the present application is to provide a new measure for treating or preventing presbyopia, which is a very interesting challenge.

#### Solution to Problem

With respect to the therapeutic effect of the lipoic acid on presbyopia found in Patent Document 1, the present inventors examined the effects of prodrugs in which the carboxylic acid portion of lipoic acid was variously modified. As a result of intensive research to solve the above problem, surprisingly, the present inventors have found ester-type prodrugs having specified structures that significantly improves the lens elasticity as compared with the above-mentioned lipoic acid choline ester, which is being clinically developed, and have reached the invention of the present disclosure. The present inventors have also found that lipoic acid prodrugs having these specified structures exhibit a high penetration property into the lens. Specifically, the present disclosure provides the following aspects of the invention.

[1] An agent/composition for treating or preventing presbyopia, comprising a compound of Formula [I] and/or Formula [II]:

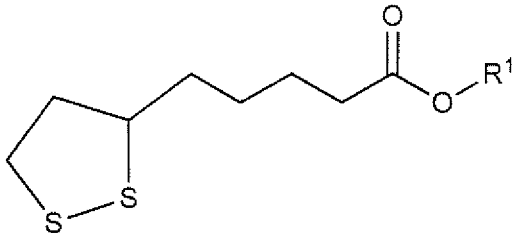

[I]

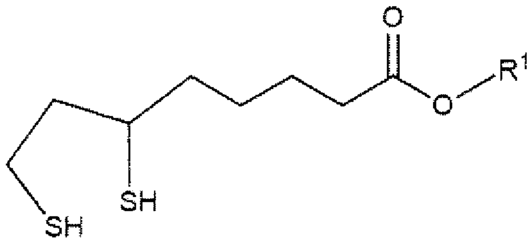

[II]

or a pharmaceutically acceptable salt thereof as an active ingredient, wherein

$R^1$ is selected from:

$C_{2-8}$ alkenyl which may be substituted with the same or different 1 to 4 (preferably 1 to 3, more preferably 1 to 2, even more preferably 1) $R^2$,
$C_{2-8}$ alkynyl which may be substituted with the same or different 1 to 4 (preferably 1 to 3, more preferably 1 to 2, even more preferably 1) $R^2$,
$C_{1-4}$ alkyl substituted with the same or different one or two 4- to 7-membered saturated heteromonocyclic groups, wherein the saturated heteromonocyclic group comprises a carbon atom and the same or different 1 to 3 heteroatoms selected from N, O and $S(O)_{0-2}$ and may be substituted with the same or different 1 to 4 (for example, 1 to 3, 1 to 2, or 1) $R^3$, and
$C_{1-4}$ alkyl substituted with the same or different one or two 9- to 10-membered bicyclic fused heterocyclic groups, wherein the bicyclic fused heterocyclic group comprises a carbon atom and the same or different 1 to 4 heteroatoms selected from N, O and $S(O)_{0-2}$ and may be substituted with the same or different 1 to 4 (for example, 1 to 3, 1 to 2, or 1) $R^3$;

$R^2$, at each occurrence, is independently selected from -OH, halo, and $C_{1-3}$ alkoxy;
$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and
$R^4$, at each occurrence, is independently selected from -OH and halo.

[2] An agent/composition for treating or preventing an eye disease accompanied by a decrease in lens elasticity, comprising a compound of Formula [I] and/or Formula [II]:

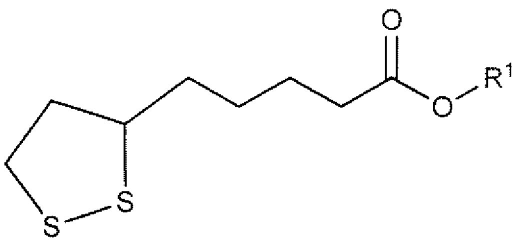

[I]

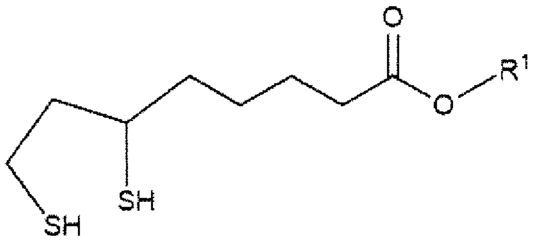

[II]

wherein $R^1$ is as defined in [1] or a pharmaceutically acceptable salt thereof as an active ingredient.

[3] The agent/composition according to [2], wherein the eye disease is accompanied by a decrease in accommodative function of the eye.

[4] An agent/composition for treating or preventing an eye disease accompanied by a decrease in accommodative function of the eye, comprising a compound of Formula [I] and/or Formula [II]:

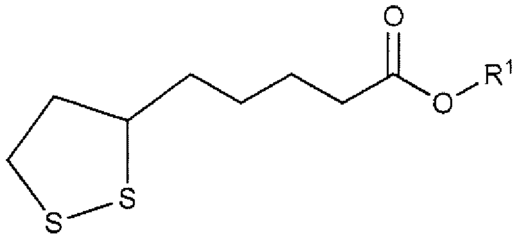

[I]

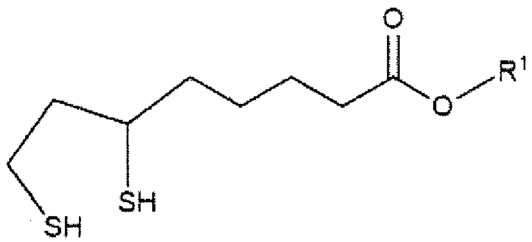

[II]

wherein $R^1$ is as defined in [1] or a pharmaceutically acceptable salt thereof as an active ingredient.

[5] The agent/composition according to any one of [2] to [4], wherein the eye disease is presbyopia.

[6] A composition comprising a compound of Formula [I] and/or Formula [II]:

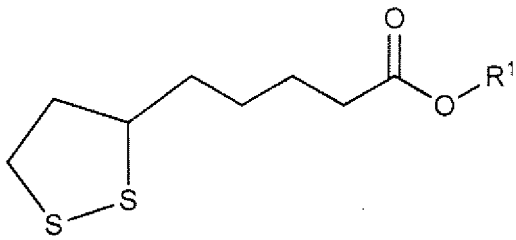

[I]

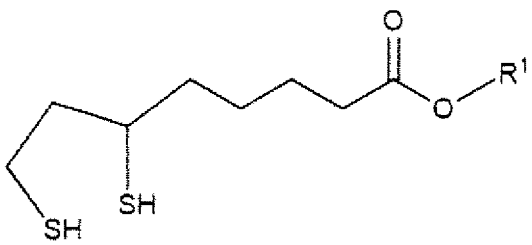

[II]

wherein $R^1$ is as defined in [1]

or a pharmaceutically acceptable salt thereof,
wherein the composition has a high penetration property into lens.

[7] The agent/composition according to any one of [1] to [6], comprising a compound of Formula [I-a] and/or Formula [II-a] :

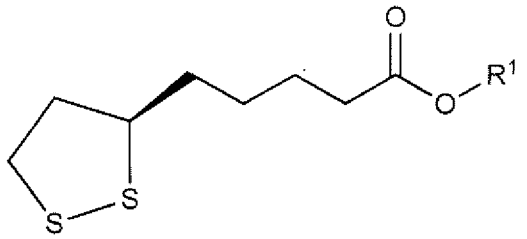

[I-a]

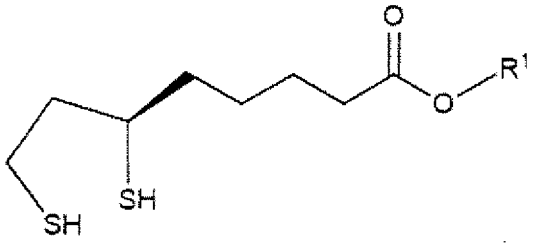

[II-a]

wherein $R^1$ is as defined in [1]
or a pharmaceutically acceptable salt thereof as an active ingredient.

[8] The agent/composition according to any one of [1] to [7], wherein

$R^1$ is selected from:

$C_{2-8}$ alkenyl which may be substituted with the same or different 1 to 4 (preferably 1 to 3, more preferably 1 to 2, even more preferably 1) $R^2$,
$C_{2-8}$ alkynyl which may be substituted with the same or different 1 to 4 (preferably 1 to 3, more preferably 1 to 2, even more preferably 1) $R^2$, and
$-(CH_2)_{1-4}$-4- to 7-membered saturated heteromonocyclyl, wherein the saturated heteromonocyclyl comprises a carbon atom and the same or different 1 to 3 heteroatoms selected from N, O and $S(O)_{0-2}$ and may be substituted with the same or different 1 to 4 (preferably 1 to 3, more preferably 1 to 2, even more preferably 1) $R^3$;

$R^2$, at each occurrence, is independently selected from -OH, halo, and $C_{1-3}$ alkoxy;
$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and
$R^4$, at each occurrence, is independently selected from -OH and halo.

[9] The agent/composition according to any one of [1] to [8], wherein

$R^1$ is selected from:

$C_{2-8}$ alkenyl which may be substituted with the same or different 1 to 4 (preferably 1 to 3, more preferably 1 to 2, even more preferably 1) $R^2$, and
$C_{2-8}$ alkynyl which may be substituted with the same or different 1 to 4 (preferably 1 to 3, more preferably 1 to 2, even more preferably 1) $R^2$; and

$R^2$, at each occurrence, is independently selected from -OH, halo, and $C_{1-3}$ alkoxy.

[10] The agent/composition according to any one of [1] to [8], wherein

$R^1$ is $C_{1-4}$ alkyl substituted with the same or different one or two 4- to 7-membered saturated heteromonocyclic groups, wherein the saturated heteromonocyclic group comprises a carbon atom and the same or different 1 to 3 heteroatoms selected from N, O and $S(O)_{0-2}$ and may be substituted with the same or different 1 to 4 (for example, 1 to 3, 1 to 2, or 1) $R^3$;

$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and

$R^4$, at each occurrence, is independently selected from -OH and halo.

[11] The agent/composition according to any one of [1] to [8], wherein

$R^1$ is $C_{1-4}$ alkyl substituted with the same or different one or two 9- to 10-membered bicyclic fused heterocyclic groups, wherein the bicyclic fused heterocyclic group comprises a carbon atom and the same or different 1 to 4 heteroatoms selected from N, O and $S(O)_{0-2}$ and may be substituted with the same or different 1 to 4 (for example, 1 to 3, 1 to 2, or 1) $R^3$;

$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and

$R^4$, at each occurrence, is independently selected from -OH and halo.

[12] The agent/composition according to any one of [1] to [8] and [10], wherein

$R^1$ is $-(CH_2)_{1-4}$-4- to 7-membered saturated heteromonocyclyl, wherein the saturated heteromonocyclyl comprises a carbon atom and the same or different 1 to 3 heteroatoms selected from N, O and $5(O)_{0-2}$ and may be substituted with the same or different 1 to 4 (preferably 1 to 3, more preferably 1 to 2, even more preferably 1) $R^3$;

$R^3$ is selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and

$R^4$, at each occurrence, is independently selected from -OH and halo.

[13] The agent/composition according to any one of [1] to [12], comprising as an active ingredient a compound selected from:

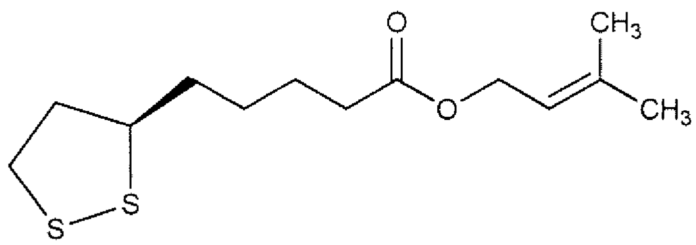

,

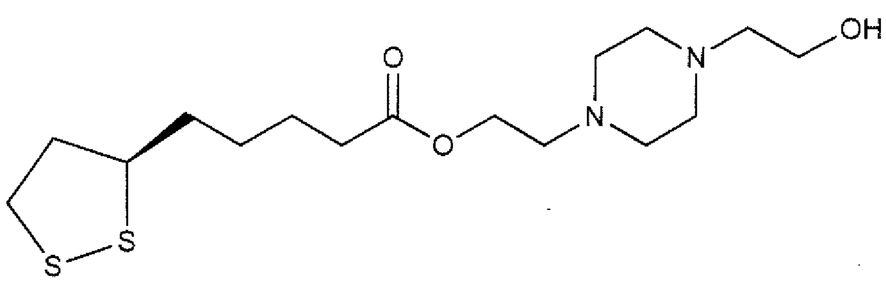

,

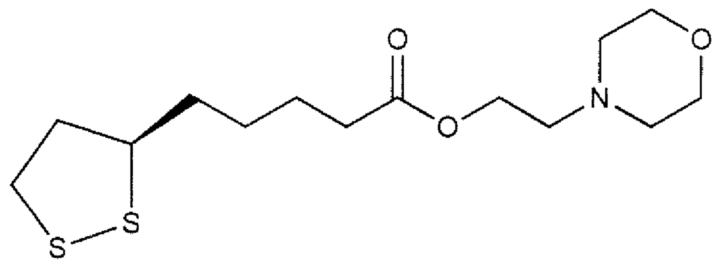

,

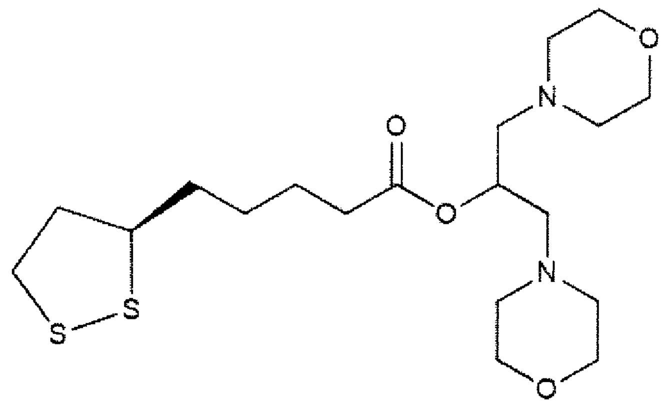

,

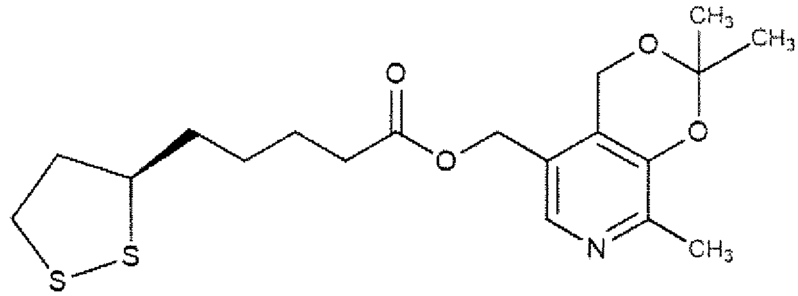

,

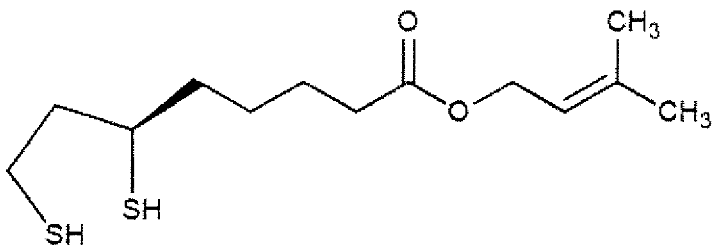

,

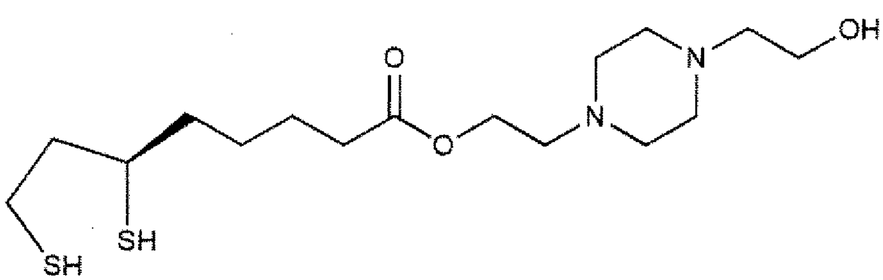

,

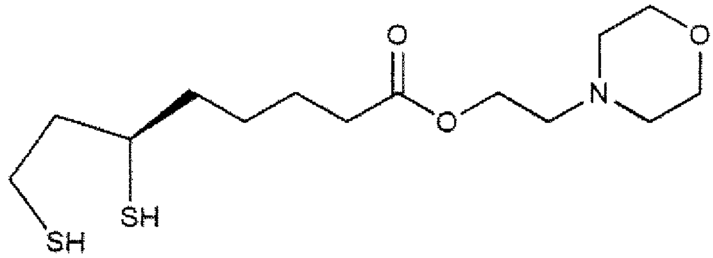

,

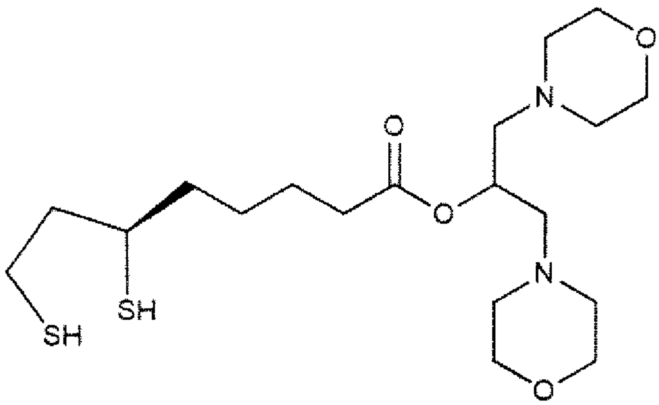

,

and

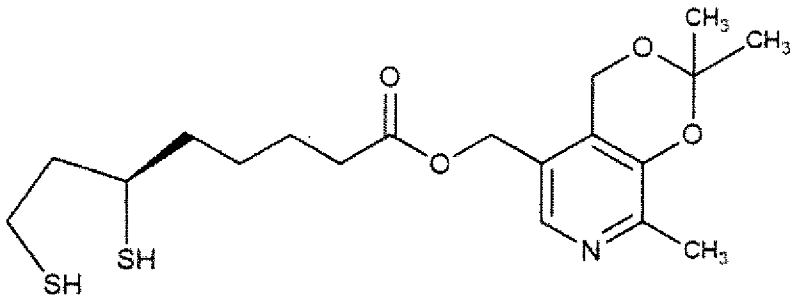

,

or a pharmaceutically acceptable salt thereof.

[14] The agent/composition according to any one of [1] to [13], wherein the agent/composition is for ophthalmic administration.

[15] The agent/composition according to any one of [1] to [14], wherein the agent/composition is an eye drop or an eye ointment.

[16] The agent/composition according to any one of [1] to [15], wherein the amount of the active ingredient comprised in the agent/composition is 0.00001 to 10%(w/v).

[17] Use of a compound of Formula [I] or Formula [II]:

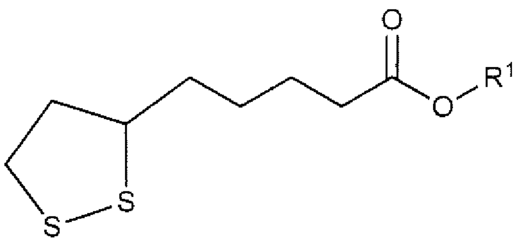

[I]

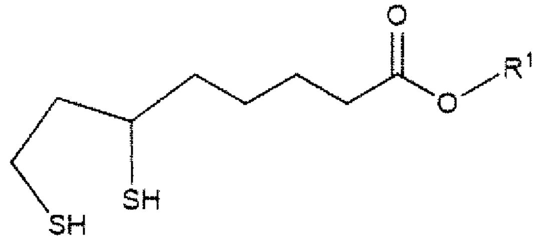

[II]

wherein $R^1$ is as defined in [1] or a pharmaceutically acceptable salt thereof, in the manufacture of an agent/composition for treating or preventing presbyopia, an eye disease accompanied by a decrease in lens elasticity, or an eye disease accompanied by a decrease in accommodative function of the eye.

[18] A compound of Formula [I] or Formula [II]:

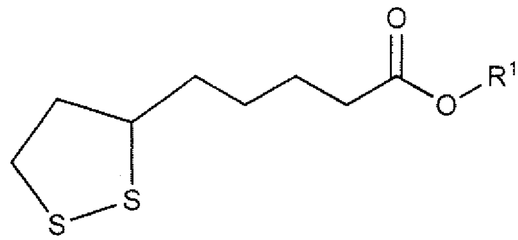

[I]

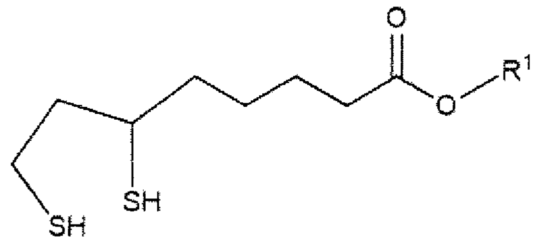

[II]

wherein $R^1$ is as defined in [1] or a pharmaceutically acceptable salt thereof, for use in the treatment or prevention of presbyopia, an eye disease accompanied by a decrease in lens elasticity, or an eye disease accompanied by a decrease in accommodative function of the eye.

[19] A method for treating or preventing presbyopia, an eye disease accompanied by a decrease in lens elasticity, or an eye disease accompanied by a decrease in accommodative function of the eye, comprising administering to a subject in need thereof an effective amount of a compound of Formula [I] and/or Formula [II]:

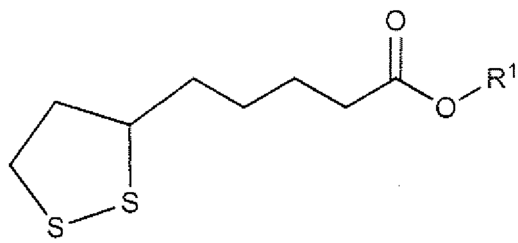

[I]

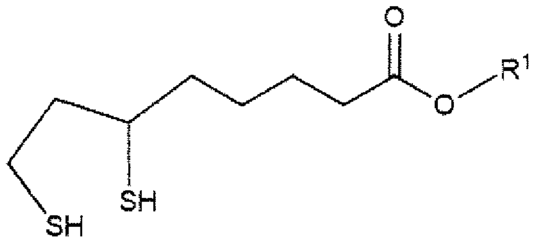

[II]

wherein $R^1$ is as defined in [1] or a pharmaceutically acceptable salt thereof.

[20] A compound of Formula [I] or Formula [II]:

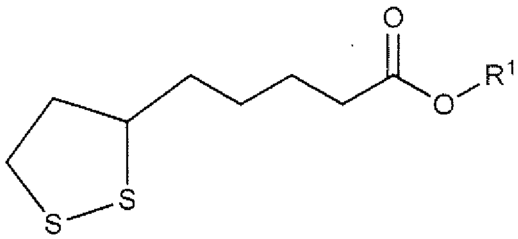

[I]

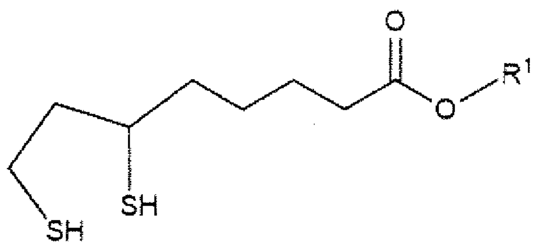

[II]

wherein $R^1$ is as defined in [1] or a pharmaceutically acceptable salt thereof, for use as a prodrug of lipoic acid.

[21] A method for improving penetration of lipoic acid into lens comprising making lipoic acid into a compound of Formula [I] or Formula [II]:

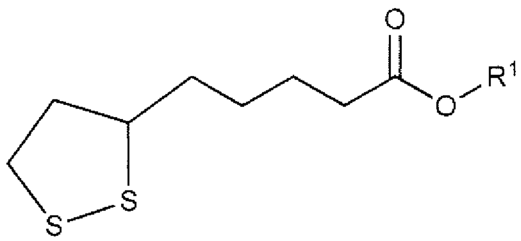

[I]

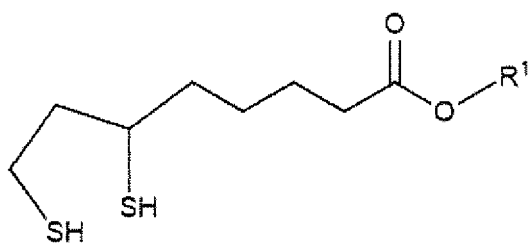

[II]

wherein $R^1$ is as defined in [1] or a pharmaceutically acceptable salt thereof.

[22] A compound of

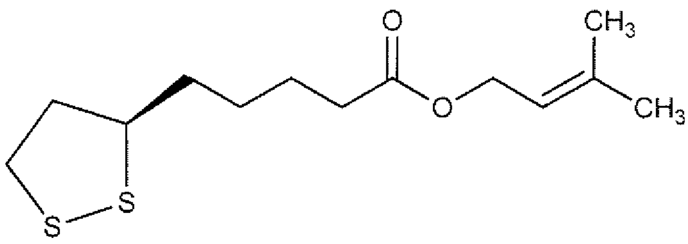

or a pharmaceutically acceptable salt thereof.

[23] A compound of

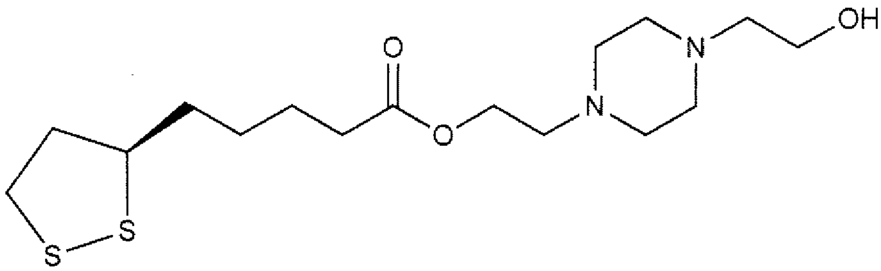

or a pharmaceutically acceptable salt thereof.

[24] A compound of

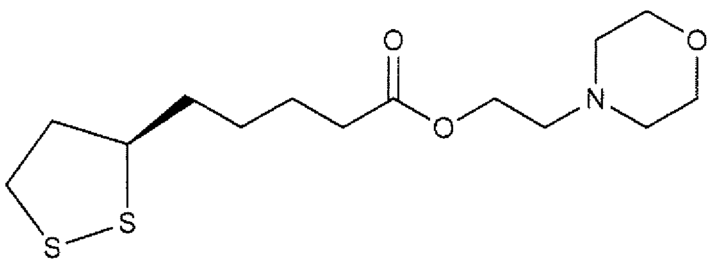

or a pharmaceutically acceptable salt thereof.

[25] A compound of

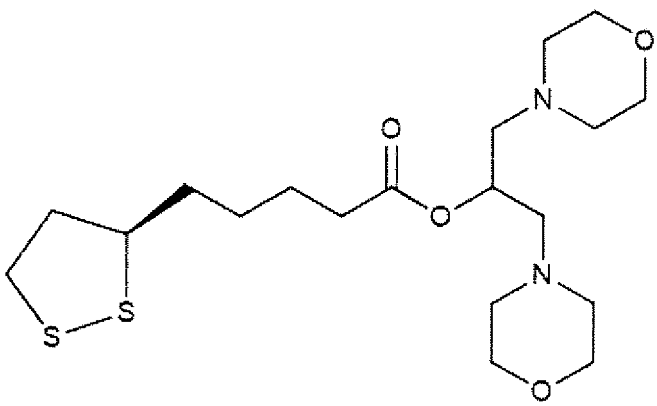

or a pharmaceutically acceptable salt thereof.

[26] A compound of

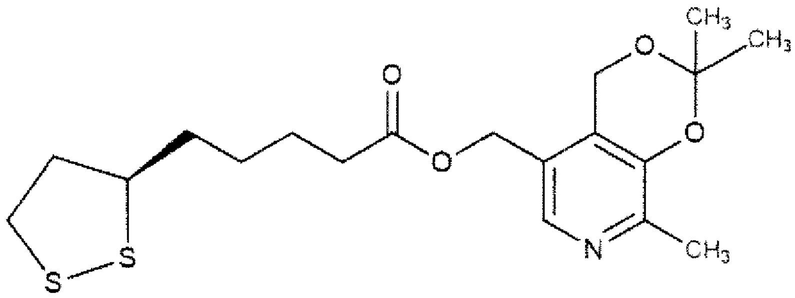

or a pharmaceutically acceptable salt thereof.

[27] A compound of

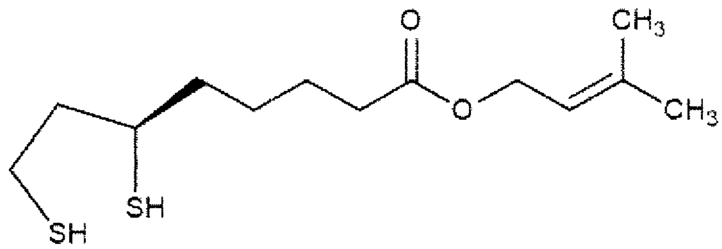

or a pharmaceutically acceptable salt thereof.

[28] A compound of

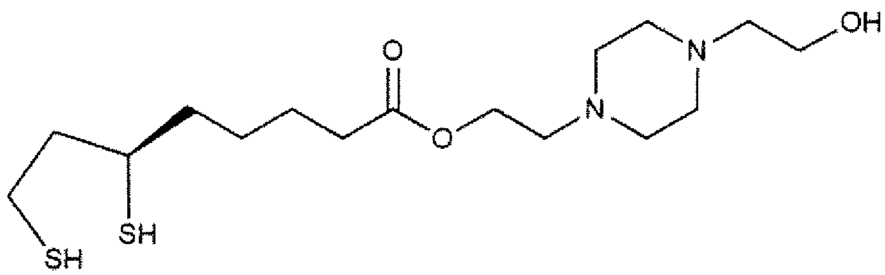

or a pharmaceutically acceptable salt thereof.

[29] A compound of

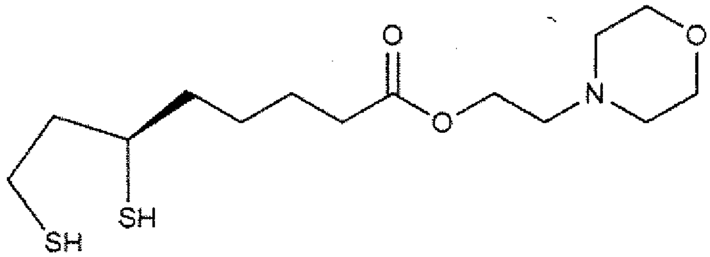

or a pharmaceutically acceptable salt thereof.

[30]

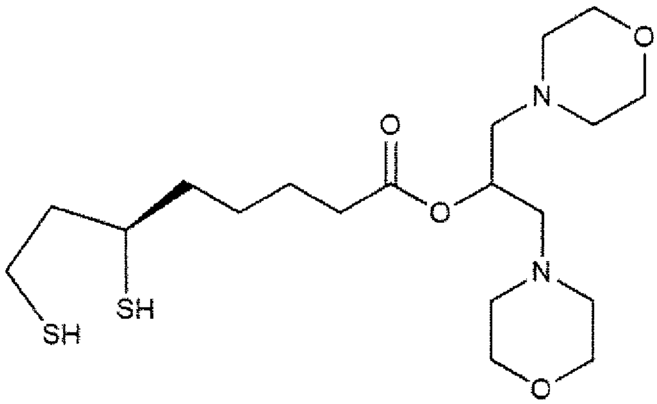

[31] A compound of

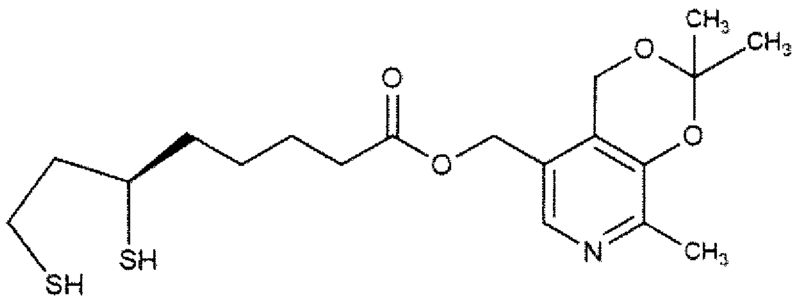

or a pharmaceutically acceptable salt thereof.

Each of the elements described in the above [1] to [31] may be optionally selected and combined.

Advantageous Effects of Invention

The composition of the present disclosure can improve the lens elasticity, which is important for lens thickness adjustment, and is therefore useful in the treatment or prevention of eye diseases such as presbyopia etc.

Description of Embodiments

Embodiments of the present invention are described in detail below.

The present disclosure provides a lipoic acid prodrug of Formula [I] or Formula [II]:

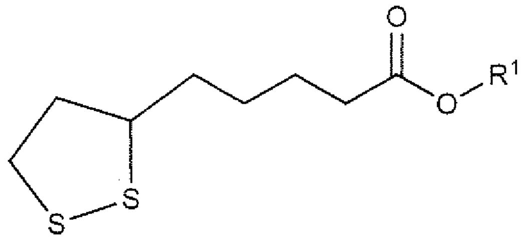

[I]

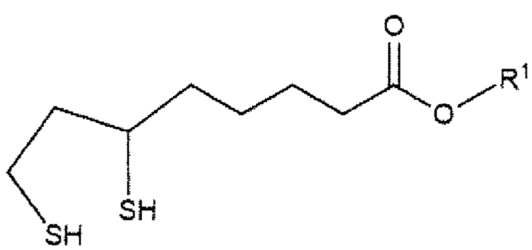

[II]

or a pharmaceutically acceptable salt thereof (hereinafter sometimes referred to as "the compound of the present invention"), wherein

$R^1$ is selected from:

$C_{2-8}$ alkenyl which may be substituted with the same or different 1 to 4 (preferably 1 to 3, more preferably 1 to 2, even more preferably 1) $R^2$,
$C_{2-8}$ alkynyl which may be substituted with the same or different 1 to 4 (preferably 1 to 3, more preferably 1 to 2, even more preferably 1) $R^2$,
$C_{1-4}$ alkyl substituted with the same or different one or two 4- to 7-membered saturated heteromonocyclic groups, wherein the saturated heteromonocyclic group comprises a carbon atom and the same or different 1 to 3 heteroatoms selected from N, O and $S(O)_{0-2}$ and may be substituted with the same or different 1 to 4 (for example, 1 to 3, 1 to 2, or 1) $R^3$, and
$C_{1-4}$ alkyl substituted with the same or different one or two 9- to 10-membered bicyclic fused heterocyclic groups, wherein the bicyclic fused heterocyclic group comprises a carbon atom and the same or different 1 to 4 heteroatoms selected from N, O and $S(O)_{0-2}$ and may be substituted with the same or different 1 to 4 (for example, 1 to 3, 1 to 2, or 1) $R^3$;

$R^2$, at each occurrence, is independently selected from -OH, halo, and $C_{1-3}$ alkoxy;
$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$;
$R^4$, at each occurrence, is independently selected from -OH, and halo.

One embodiment of the compound of the present invention includes a compound of Formula [I-a] or Formula [II-a]:

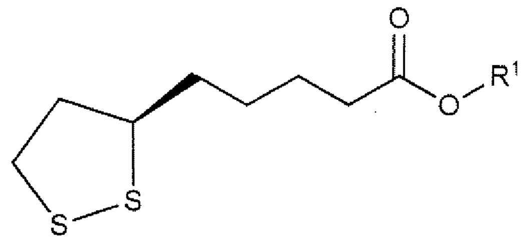

[I-a]

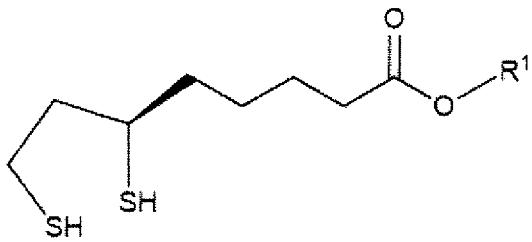

[II-a]

wherein $R^1$ is as defined in the above Formula [I]
or a pharmaceutically acceptable salt thereof.
In one embodiment of the compound of the present invention,

$R^1$ is selected from:

$C_{2-8}$ alkenyl (for example, $C_{3-7}$ alkenyl, $C_{4-6}$ alkenyl, $C_5$ alkenyl) which may be substituted with the same or different 1 to 4 (preferably 1 to 3, more preferably 1 to 2, even more preferably 1) $R^2$,
$C_{2-8}$ alkynyl (for example, $C_{3-7}$ alkynyl, $C_{4-6}$ alkynyl, $C_5$ alkynyl) which may be substituted with the same or different 1 to 4 (preferably 1 to 3, more preferably 1 to 2, even more preferably 1) $R^2$, and
$-(CH_2)_{1-4}$-4- to 7-membered saturated heteromonocyclyl, wherein the saturated heteromonocyclyl comprises a carbon atom and the same or different 1 to 3 heteroatoms selected from N, O and $S(O)_{0-2}$ and may be

substituted with the same or different 1 to 4 (preferably 1 to 3, more preferably 1 to 2, even more preferably 1) $R^3$;
$R^2$, at each occurrence, is independently selected from -OH, halo, and $C_{1-3}$ alkoxy;

$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$;
$R^4$, at each occurrence, is independently selected from -OH and halo.

In one embodiment of the compound of the present invention,

$R^1$ is selected from:

$C_{2-8}$ alkenyl (for example, $C_{3-7}$ alkenyl, $C_{4-6}$ alkenyl, $C_5$ alkenyl) which may be substituted with the same or different 1 to 4 (preferably 1 to 3, more preferably 1 to 2, even more preferably 1) $R^2$, and
$C_{2-8}$ alkynyl (for example, $C_{3-7}$ alkynyl, $C_{4-6}$ alkynyl, $C_5$ alkynyl) which may be substituted with the same or different 1 to 4 (preferably 1 to 3, more preferably 1 to 2, even more preferably 1) $R^2$; and

$R^2$, at each occurrence, is independently selected from -OH, halo, and $C_{1-3}$ alkoxy.

In one embodiment of the compound of the present invention,

$R^1$ is $C_{2-8}$ alkenyl (for example, $C_{3-7}$ alkenyl, $C_{4-6}$ alkenyl, $C_5$ alkenyl) which may be substituted with the same or different 1 to 4 (preferably 1 to 3, more preferably 1 to 2, even more preferably 1) $R^2$; and
$R^2$, at each occurrence, is independently selected from -OH, halo, and $C_{1-3}$ alkoxy.

In one embodiment of the compound of the present invention,

$R^1$ is $C_{2-8}$ alkynyl (for example, $C_{3-7}$ alkynyl, $C_{4-6}$ alkynyl, $C_5$ alkynyl) which may be substituted with the same or different 1 to 4 (preferably 1 to 3, more preferably 1 to 2, even more preferably 1) $R^2$; and
$R^2$, at each occurrence, is independently selected from -OH, halo, and $C_{1-3}$ alkoxy.

In one embodiment of the compound of the present invention,

$R^1$ is $C_{1-4}$ alkyl substituted with the same or different one or two 4- to 7-membered saturated heteromonocyclic groups, wherein the saturated heteromonocyclic group comprises a carbon atom and the same or different 1 to 3 heteroatoms selected from N, O and $5(O)_{0-2}$ and may be substituted with the same or different 1 to 4 (for example, 1 to 3, 1 to 2, or 1) $R^3$;
$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and
$R^4$, at each occurrence, is independently selected from -OH and halo.

In one embodiment of the compound of the present invention,

$R^1$ is $C_{1-4}$ alkyl substituted with the same or different one or two 4- to 7-membered saturated heteromonocyclic groups, wherein the saturated heteromonocyclic group is selected from piperazinyl (for example, 1-piperazinyl) and morpholinyl (for example, 4-morpholinyl) and may be substituted with the same or different 1 to 4 (for example, 1 to 3, 1 to 2, or 1) $R^3$;
$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and
$R^4$, at each occurrence, is independently selected from -OH and halo.

In one embodiment of the compound of the present invention,

$R^1$ is $C_{1-4}$ alkyl substituted with the same or different 1 or 2 (preferably 1) 9- to 10-membered bicyclic fused heterocyclic

groups, wherein the bicyclic fused heterocyclic group comprises a carbon atom and the same or different 1 to 4 heteroatoms selected from N, O and $S(O)_{0-2}$ and may be substituted with the same or different 1 to 4 (for example, 1 to 3, 1 to 2, or 1) $R^3$;

$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and

$R^4$, at each occurrence, is independently selected from -OH and halo.

In one embodiment of the compound of the present invention,

$R^1$ is $C_{1-4}$ alkyl substituted with the same or different 1 or 2 (preferably 1) 9- to 10-membered bicyclic fused heterocyclic groups, wherein the bicyclic fused heterocyclic group is selected from

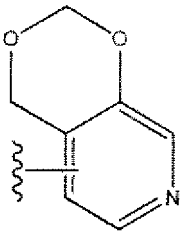 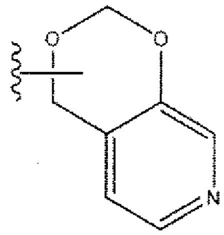 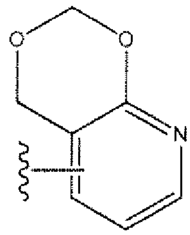 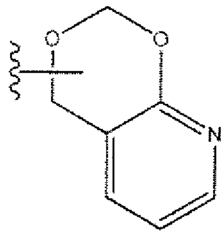

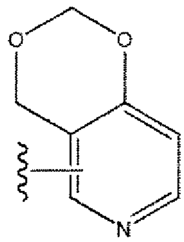 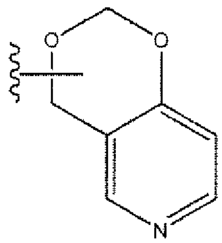 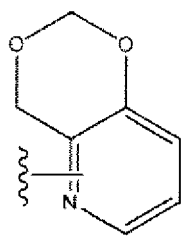 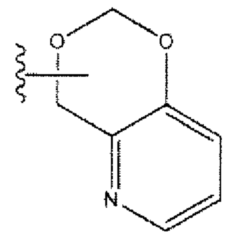

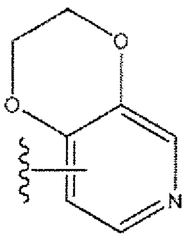 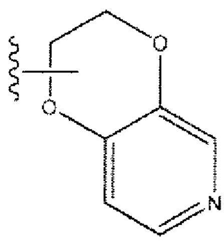 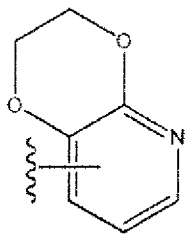 and 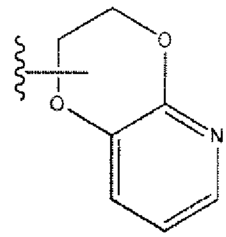

(preferably

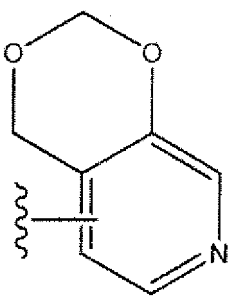
)

and may be substituted with the same or different 1 to 4 (for example, 1 to 3, 1 to 2, or 1) $R^3$ ;

$R^3$, at each occurrence, is independently selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1)

$R^4$; and

$R^4$, at each occurrence, is independently selected from -OH and halo.

In one embodiment of the compound of the present invention,

$R^1$ is $-(CH_2)_{1-4}$-4- to 7-membered saturated heteromonocyclyl, wherein the saturated heteromonocyclyl comprises a carbon atom and the same or different 1 to 3 heteroatoms selected from N, O and $S(O)_{0-2}$ (for example, the saturated heteromonocyclyl is selected from piperazinyl (for example, 1-piperazinyl) and morpholinyl(for example, 4-morpholinyl) and may be substituted with the same or different 1 to 4 (preferably 1 to 3, more preferably 1 to 2, even more preferably 1) $R^3$;

$R^3$ is selected from -OH, halo, $C_{1-3}$ alkyl which may be substituted with the same or different 1 to 4 (preferably 1 to 3, more preferably 1 to 2, even more preferably 1) $R^4$, and $C_{1-3}$ alkoxy which may be substituted with the same or different 1 to 4 (preferably 1 to 3, more preferably 1 to 2, even more preferably 1) $R^4$; and

$R^4$, at each occurrence, is independently selected from -OH and halo.

One embodiment of the compound of the present invention includes a compound or a pharmaceutically acceptable salt thereof, wherein said compound is selected from

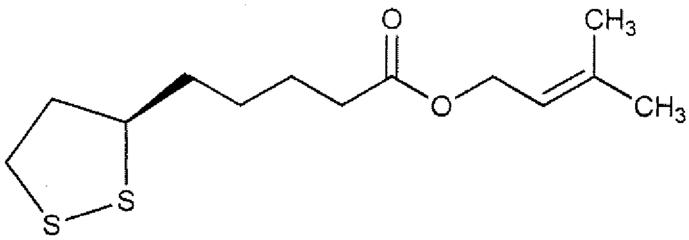

,

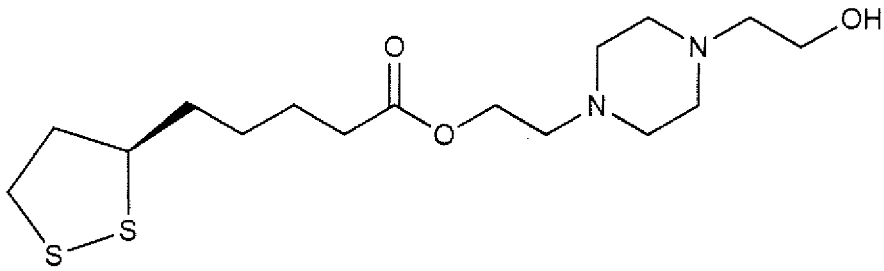

,

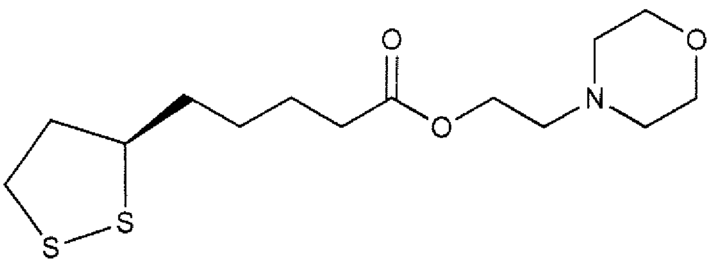

,

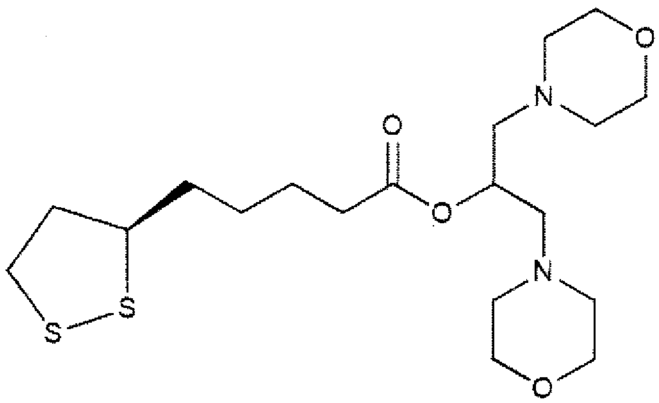

,

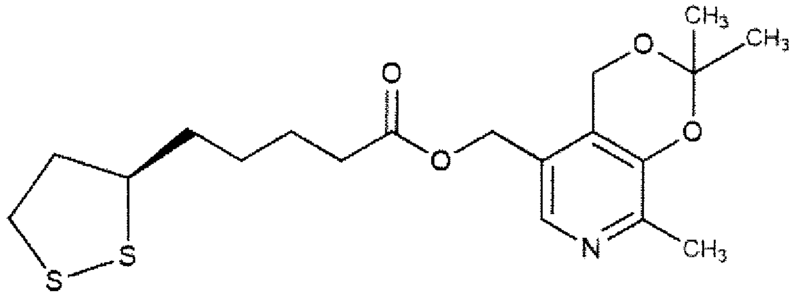

,

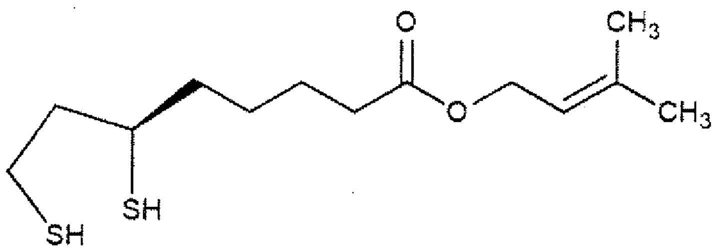

,

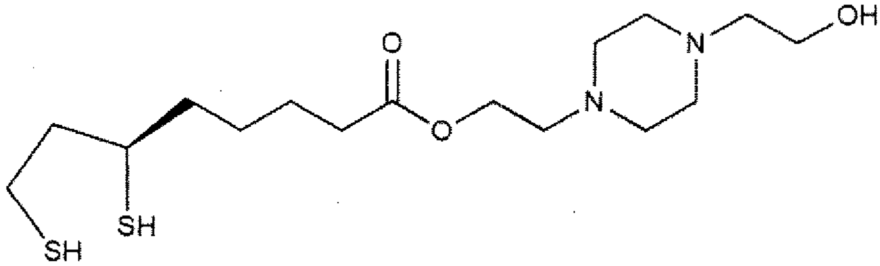

,

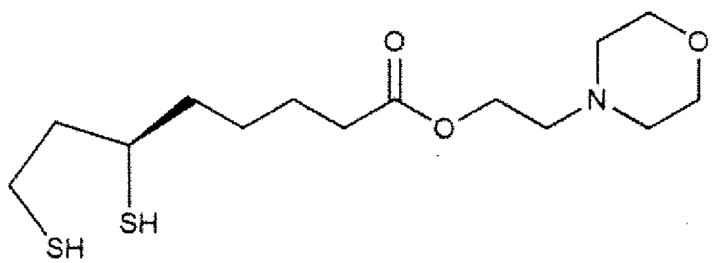

,

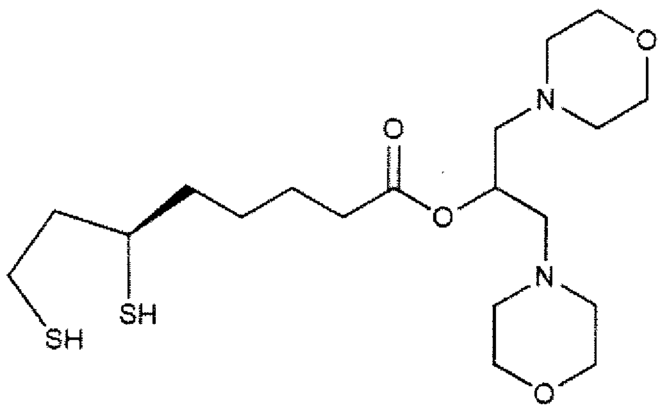

,

and

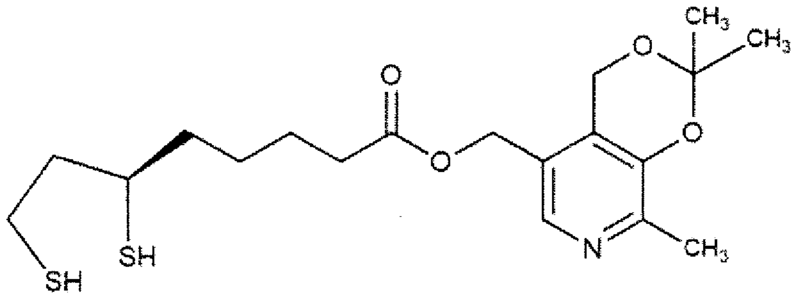

.

As described above, the compound of the present invention may be a lipoic acid prodrug as represented by Formula [I] having a 1,2-dithiolane ring or a pharmaceutically acceptable salt thereof and may also be a lipoic acid prodrug as represented by Formula [II] having a 1,3-propanedithiol moiety in which the 1,2-dithiolane ring is opened or a pharmaceutically acceptable salt thereof, because the 1,2-dithiolane ring and 1,3-propanedithiol moiety may be mutually converted in vivo.

The present disclosure further provides an agent/composition (hereinafter sometimes referred to as "the agent/composition of the present invention") comprising as an active ingredient lipoic acid prodrug(s) of the above Formula [I] and/or the above Formula [II] or a pharmaceutically acceptable salt thereof. The compound/agent/composition of the present invention may be used for treating or preventing presbyopia. The compound/agent/composition of the present invention may be used to improve lens elasticity. In addition, the compound/agent/composition of the present invention may be used to improve eye accommodation. Further, the compound of the present invention may efficiently penetrate to the lens compared to lipoic acid, and may be used as a lipoic acid prodrug having a high penetration property into lens. The agent/composition of the present invention may be used for efficiently delivering the active metabolite lipoic acid to the lens.

In the present disclosure, "lipoic acid prodrug" refers to a compound that is likely to be metabolized in vivo to produce the active metabolite lipoic acid, which may or may not be verified to be actually produced in vivo. For example, a lipoic acid prodrug wherein the carboxylic acid part of lipoic acid is esterified is exemplified.

In the present disclosure, "$C_{2-8}$ alkenyl" means a straight or branched-chain hydrocarbon group having 2 to 8 carbon atoms and comprising at least one double bond. Examples of $C_{2-8}$ alkenyl include alkenyl having 3 to 7 carbon atoms ($C_{3-7}$ alkenyl), alkenyl having 4 to 6 carbon atoms ($C_{4-6}$ alkenyl), alkenyl having 5 carbon atoms ($C_5$ alkenyl). Specific examples of $C_{2-8}$ alkenyl include vinyl, 1-propenyl, 2-propenyl, isopropenyl, 1-butenyl, 2-butenyl, 3-butenyl, 1-methyl-1-propenyl, 1-methyl-2-propenyl, 2-methyl-1-propenyl, 2-methyl-2-propenyl, 1-ethylvinyl, 1-pentenyl, 2-pentenyl, 3-pentenyl, 4-pentenyl, 1,2-dimethyl-1-propenyl, 1,2-dimethyl-2-propenyl, 1-ethyl-1-propenyl, 1-ethyl-2-propenyl, 1-methyl-1-butenyl, 1-methyl-2-butenyl, 2-methyl-1-butenyl, 3-methyl-2-butenyl, 1-isopropylvinyl, 2,4-pentadienyl, 1-hexenyl, 2-hexenyl, 3-hexenyl, 4-hexenyl, 5-hexenyl, 2,4-hexadienyl, 1-methyl-1-pentenyl, 3,3-dimethylbutenyl (i.e., 3,3-dimethylbut-1-enyl) and the like.

In the present disclosure, "$C_{2-8}$ alkynyl" means a straight or branched-chain hydrocarbon group having 2 to 8 carbon atoms and comprising at least one triple bond. Examples of $C_{2-8}$ alkynyl include alkynyl having 3 to 7 carbon atoms ($C_{3-7}$ alkynyl), alkynyl having 4 to 6 carbon atoms ($C_{4-6}$ alkynyl), alkynyl having 5 carbon atoms ($C_5$ alkynyl). Specific examples of "$C_{2-8}$ alkynyl" include ethynyl, 1-propynyl, 2-propynyl, isopropynyl, 1-butynyl, 2-butynyl, 3-butynyl,

1-methyl-1-propynyl, 1-methyl-2-propynyl, 2-methyl-2-propynyl, 1-ethylethynyl, 3,3-dimethylbutynyl, and the like.

In the present disclosure, "halo" means fluoro, chloro, bromo or iodo.

In the present disclosure, "$C_{1-3}$ alkoxy" means a straight or branched-chain alkoxy having 1 to 3 carbon atoms. Examples of "$C_{1-3}$ alkoxy" include methoxy, ethoxy, propoxy, and isopropoxy.

In the present disclosure, "$C_{1-4}$ alkyl" means a straight or branched-chain saturated hydrocarbon group having 1 to 4 carbon atoms. Examples of "$C_{1-4}$ alkyl" include methyl, ethyl, propyl, isopropyl, butyl, isobutyl, s-butyl, and t-butyl.

In the present disclosure, "$C_{1-3}$ alkyl" means a straight or branched-chain saturated hydrocarbon group having 1 to 3 carbon atoms. Examples of "$C_{1-3}$ alkyl" include methyl, ethyl, propyl, isopropyl.

In the present disclosure, "4- to 7-membered saturated heteromonocyclic group" (including "4- to 7-membered saturated heteromonocyclyl" in the $(CH_2)_{1-4}$-4- to 7-membered saturated heteromonocyclyl") means a monocyclic saturated heterocyclic group comprising carbon atom(s) and the same or different 1 to 3 (preferably 1 to 2) heteroatoms selected from N, O and $S(O)_{0-2}$, wherein the number of atoms constituting the ring is 4 to 7 (preferably 5 to 7, more preferably 6). The "4- to 7-membered saturated heteromonocyclic group" may have a bond at any of carbon atom(s) and nitrogen atom(s) constituting the ring. Examples of the "4- to 7-membered saturated heteromonocyclic group" include azetidinyl, pyrrolidinyl, piperidinyl, piperazinyl (for example, 1-piperazinyl), azepanyl, morpholinyl (for example, 4-morpholinyl), thiomorpholinyl, dioxothiomorpholinyl, oxazolidinyl, thiazolidinyl, imidazolidinyl, oxazolidinyl, tetrahydropyranyl, tetrahydrofuryl, thianyl, and dioxothianyl. Preferred examples of the 4- to 7-membered saturated heteromonocyclic group include piperazinyl (for example, 1-piperazinyl) and morpholinyl (for example, 4-morpholinyl).

Examples of "$-(CH_2)_{1-4}$-4- to 7-membered saturated heteromonocyclyl" include $-(CH_2)$-4- to 7-membered saturated heteromonocyclyl, $-(CH_2)_2$-4- to 7-membered saturated heteromonocyclyl, $-(CH_2)_3$-4- to 7-membered saturated heteromonocyclyl, and $-(CH_2)_4$-4- to 7-membered saturated heteromonocyclyl.

In the present disclosure, "9- to 10-membered bicyclic fused heterocyclic group" means a bicyclic fused ring which comprises carbon atoms and the same or different 1 to 4 (for example, 1 to 3, 1 to 2, or 1) heteroatoms selected from N, O and $S(O)_{0-2}$, wherein the number of atoms constituting the ring is 9 to 10 (preferably 10). The "9-to 10-membered bicyclic fused heterocyclic group" may have a bond at any of carbon atom(s) and nitrogen atom(s) constituting the ring. Examples of the "9- to 10-membered bicyclic fused heterocyclic group" include indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzotriazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, pyrazolopyridine, triazolopyridyl, imidazothiazolyl, pyrazinopyridazinyl, dihydrobenzofuryl, tetrahydroquinolyl, tetrahydroisoquinolyl, and tetrahydrobenzothienyl.

Other examples of the "9- to 10-membered bicyclic fused heterocyclic group" include groups represented by the following formulae. In the following formulae, a bond traversing an ring means that the bond may be located at any of the available positions of the ring.

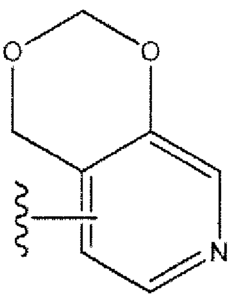 , 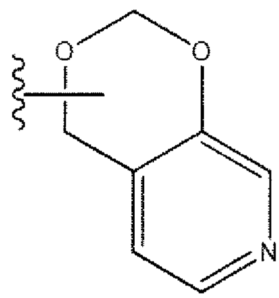 , 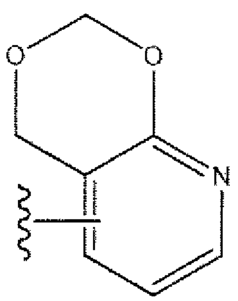 ,

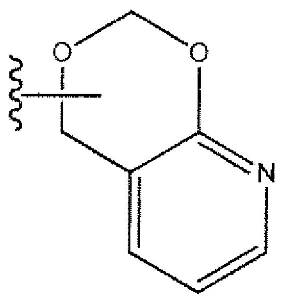 , 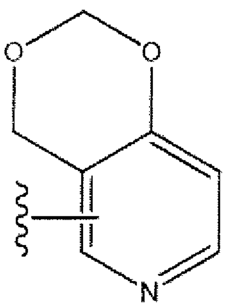 , 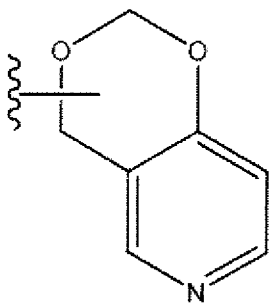 , 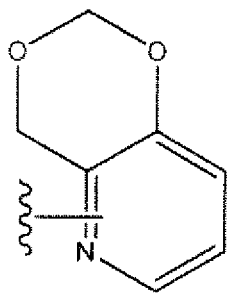 ,

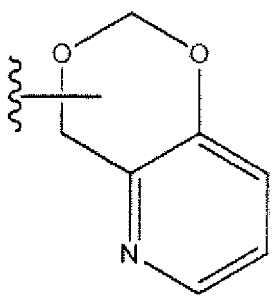
,
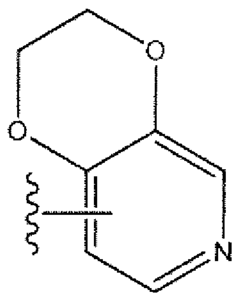
,
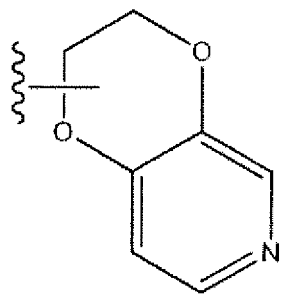
,
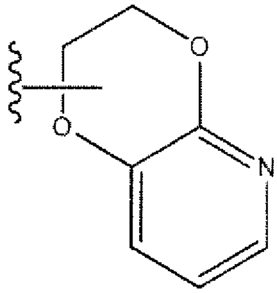
,
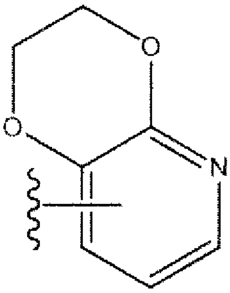
.

In the compound of the present invention, pharmaceutically acceptable salts are not particularly limited as long as they are pharmaceutically acceptable salts. Examples of pharmaceutically acceptable salts include, inorganic salts such as hydrochlorides, hydrobromides, hydroiodides, nitrates, sulfates, phosphates, etc.; organic acid salts such as acetates, trifluoroacetates, benzoates, oxalates, malonates, succinates, maleates, fumarates, tartrates, citrates, methanesulfonates, ethanesulfonates, trifluoromethanesulfonates, benzenesulfonates, *p*-toluenesulfonates, glutamates, aspartates, etc.; metal salts such as sodium salts, potassium salts, calcium salts, and magnesium salts, etc.; inorganic salts such as ammonium salts, etc.; and organic amine salts such as triethylamine salts, guanidine salts, etc. Examples of pharmaceutically acceptable salts include preferably sodium salts and potassium salts.

In the the present disclosure, the compound of the present invention may be in the form of hydrates or solvates.

The agent/composition of the present invention may comprise a single type or a plurality of types of the compound of the present invention.

The amount of the compound of the present invention comprised in the agent/composition of the present invention (the total amount when a plurality of the compounds of the present invention is comprised) is not particularly limited and may be selected from a wide range depending on dosage forms etc.

For example, the amount of the compound of the present invention comprised in the agent/composition of the present invention is 0.00001 to 10% (w/v), preferably 0.0001 to 5% (w/v), more preferably 0.001 to 3% (w/v), even more preferably 0.003 to 2% (w/v), even more preferably 0.01 to 2% (w/v), particularly preferably 0.03 to 1.5% (w/v), more preferably 0.03 to 0.5% (w/v), and even more preferably 0.03 to 0.3% (w/v). An example of the lower limit of the amount is 0.00001% (w/v), a preferable example is 0.0001% (w/v), a more preferable example 0.001% (w/v), a more preferable example is 0.003% (w/v), a particularly preferable example is 0.01% (w/v), a further particularly preferable example is 0.02% (w/v), and a further particularly preferable example is 0.03% (w/v). An example of the upper limit of the amount is 10% (w/v), a preferable example is 5% (w/v), a more preferable example is 3% (w/v), a particularly preferable example is 2% (w/v), a further particularly preferable example is 1.5% (w/v), and a further particularly preferable example is 1% (w/v). In particular, from the viewpoint of suppressing eye irritation, the upper limit of the amount is preferably 1.5% (w/v), more preferably 0.3% (w/v), even more preferably 0.1% (w/v). A preferred range of the amount may be indicated by a combination of the above examples of lower and upper limits.

In addition, for example, the amount of the compound of the present invention comprised in the agent/composition of the present invention is 0.00001 to 10% (w/w), preferably 0.0001 to 5% (w/w), more preferably 0.001 to 3% (w/w), even more preferably 0.003 to 2% (w/w), even more preferably 0.01 to 2% (w/w), particularly preferably 0.03 to 1.5% (w/w), more preferably 0.03 to 0.5% (w/w), and even more preferably 0.03 to 0.3% (w/w). An example of the lower limit of the amount is 0.00001% (w/w), a preferable example is 0.0001% (w/w), a more preferable example is 0.001% (w/w), a even more preferable example is 0.0003% (w/w), a particularly preferable example is 0.01% (w/w), a further particularly preferable example is 0.02% (w/w), and a further particularly preferable example is 0.03% (w/w). An example of the upper limit of the amount is 10% (w/w), a preferable example is 5% (w/w), a more preferable example is 3% (w/w), a particularly preferable example is 2% (w/w), a further particularly preferable example is 1.5% (w/w), and a further particularly preferable example is 1% (w/w). In particular, from the viewpoint of suppressing eye irritation, the upper limit

of the amount is preferably 1.5% (w/w), more preferably 0.3% (w/w), even more preferably 0.1% (w/w). A preferred range of the amount may be indicated by a combination of the above examples of lower and upper limits.

In the present disclosure, "% (w/v)" means the mass (g) of the active ingredient (the compound of the present invention) or an additive (surfactant, etc.) comprised in 100 mL of an agent. For example, "0.01% (w/v) of the compound of the present invention" means that the amount of the compound of the present invention comprised in 100 mL of an agent is 0.01g.

In the present disclosure, "% (w/w)" means the mass (g) of the active ingredient (the compound of the present invention) or an additive (surfactant, etc.) comprised in 100 g of an agent. For example, "0.01% (w/w) of the compound of the present invention" means that the amount of the compound of the present invention comprised in 100 g of an agent is 0.01g.

When the compound of the present invention is in the form of salt, or in the form of hydrate or solvate (including the form of hydrate or solvate of the salt), the amount of the compound of the present invention comprised in an agent may mean the mass of the salt, hydrate, or solvate (including the hydrate or solvate of the salt) added into the agent, or may mean the mass converted as a free form of the lipoic acid prodrug, preferably may mean the mass converted as a free form of the lipoic acid prodrug.

In this disclosure, the term "presbyopia" means a symptom/disease that is determined to be presbyopia based on general criteria used by a physician or professional.

For example, diagnostic criteria for presbyopia include:

Decreased near vision is noticed as a subjective symptom in a binocular vision test, and a binocular daily life visual acuity, which is a binocular distant visual acuity measured under the same condition as daily life, is less than 0.4 at 40 cm distance(clinical presbyopia); and/or

With or without subjective symptoms, under unilateral best-correction where a corrected visual acuity of one eye is equal to or more than 1.0 (decimal visual acuity), accommodative amplitude is less than 2.5 Diopters" (medical presbyopia).

However, if an accommodometer etc. is not available, a simple criterion wherein a visual acuity at 40 cm is less than 0.4 may be used.

In the present disclosure, the term "an eye disease accompanied by a decrease in lens elasticity" refers to an eye disease considered in the field of ophthalmology to be accompanied by a decrease in lens elasticity, including, for example, presbyopia (e.g., presbyopia due to aging), and a hardening of the lens induced by drugs and the like.

In the present disclosure, the term "accommodative function of the eye" refers to an eye function that automatically focuses on distant and/or near objects. The term "an eye disease accompanied by a decrease in accommodative function of the eye" refers to an eye disease considered in the field of ophthalmology to be accompanied by a decrease in accommodative function of the eye, including, for example, presbyopia (e.g., presbyopia due to aging), and a hardening of the lens induced by drugs etc., and decreased accommodation function induced by seeing near objects for a long time.

The efficacy of the agent/composition of the present invention may be evaluated, for example, as an increase in "accommodative amplitude of the eye".

The accommodative amplitude of the eye can be measured as a Diopter (D) which can be determined by the following expression 1:

$$\text{Diopter (D)} = 1/\text{Near Point Distance (m)} \quad \text{(Expression 1)}.$$

In general, the accommodative amplitude of the eye is greater than 10 diopters at 10 years, then gradually decreases to about 3 diopters at about 45 years and is almost lost at about 60 years. When the accommodative amplitude decreases to about 3 diopters, it becomes difficult to focus on near objects (about 30 cm) in daily life, and subjective symptoms of presbyopia appear.

The efficacy of the agent/composition of the present invention may be evaluated, for example, as an improvement in "visual acuity". The visual acuity can be measured as near visual acuity (uncorrected visual acuity, distance-corrected near visual acuity, corrected visual acuity) and can be measured by using decimal visual acuity, fractional visual acuity, or logMAR.

In general, when near visual acuity which is defined to be measured at about 40 cm decreases to below 0.4, it causes difficulty in seeing near objects, and subjective symptoms of presbyopia appear. The agent/composition of the present invention may be used to improve near visual acuity (e.g., distance-corrected near visual acuity).

The agent/composition of the present invention may begin to exhibit an efficacy within one year, preferably within six months, more preferably within one month, more preferably within one week, and even more preferably within one day after the administration. Further, once an efficacy is exerted, the efficacy may be exerted continuously until

after one day, preferably until after one week, more preferably until after one month, more preferably until after six months, particularly preferably until after one year, and even more preferably until after three years.

The agent/composition of the present invention may be administered, for example, so as to increase the accommodative amplitude of the eye by at least about 0.5 diopters (preferably at least about 1 diopter, more preferably at least about 1.5 diopters, more preferably at least about 2 diopters, even more preferably at least about 3 diopters, and still more preferably at least about 4 diopters, particularly preferably at least about 5 diopters, and still more preferably at least about 10 diopters).

The agent/composition of the present invention may be administered, for example, so as to increase distance-corrected near visual acuity (DCNVA) by at least about 0.5 logMAR (preferably about at least 1.0 logMAR, more preferably about at least 1.5 logMAR, even more preferably about 2.0 logMAR, even more preferably about 3.0 logMAR, particularly preferably about 4.0 logMAR, particularly preferably about 5.0 logMAR, and even more preferably about 6.0 logMAR).

The term "distance-corrected near visual acuity" generally refers to near visual acuity measured with distance visual acuity corrected to $\leq$ 0.0 logMAR (decimal visual acuity of 1.0 or more).

The agent/composition of the present invention may be administered, for example, so as to restore the accommodative amplitude of the eye to at least about 0.5 diopters (preferably at least about 1 diopter, more preferably at least about 1.5 diopters, more preferably at least about 2 diopters, more preferably at least about 3 diopters, particularly preferably at least about 4 diopters, particularly preferably at least about 5 diopters, and still more preferably at least about 10 diopters).

The agent/composition of the present invention may be administered, for example, so as to restore the distance-corrected near visual acuity (DCNVA) to at least about 0.5 logMAR (preferably at least about 1.0 logMAR, more preferably at least about 1.5 logMAR, even more preferably about 2.0 logMAR, even more preferably about 3.0 logMAR, particularly preferably about 4.0 logMAR, particularly preferably about 5.0 logMAR, and even more preferably about 6.0 logMAR).

In the present disclosure, the treatment or prevention of presbyopia includes increasing an elasticity of the lens, improving an ability to adjust a thickness of lens, and/or improving an accommodative function of the eye.

Although subjective symptoms of presbyopia generally appear at about 45 years of age as mentioned above, age-related decline in eye accommodation has been progressing since teens. The agent/composition of the present invention may be used after the subjective symptoms of presbyopia appear, and may be used to prevent and/or delay progression of presbyopia before the subjective symptoms of presbyopia appear.

In the present disclosure, "penetration of lipoic acid into lens" refers to the ease with which lipoic acid is delivered into the lens when the compound of the present invention is administered (preferably by eye drop administration) into the body. "A high penetration property into lens" means that lipoic acid is more delivered to the lens when administered (preferably by eye drop administration) into the body as compared with lipoic acid. The method for testing whether or not "a high penetration property into lens" is shown is not particularly limited, but can be evaluated by, for example, the following pharmacokinetic study-1 or pharmacokinetic study-2.

The subjects of administration of the agent/composition of the present invention are mammals including livestock such as cattle and pigs; rabbits, monkeys, dogs, cats, and humans, preferably humans.

In this disclosure, "treatment (treating)" and "prevention (preventing)" may include, in addition to treating and preventing a disease, alleviating symptoms of the disease, delaying progression of the disease, suppressing symptoms of the disease, and inducing improvement in symptoms of the disease.

The agent/composition of the present invention may be administered orally or parenterally (e.g., ocularly, nasally, transdermally, transmucosally, by injection, etc.). From the viewpoint that the agent/composition of the present invention may be less irritating to the eye and exerts an superior effect, the agent/composition of the present invention is preferably administered into eye. The agent/composition of the present invention may be prepared in the usual manner in the art by mixing the active ingredient with, for example, one or more pharmaceutically acceptable additives, for example, in the form of oral preparations such as tablets, capsules, granules, powders, lozenges, syrups, emulsions, suspensions, and the like, or parenteral preparations such as eye drops, ophthalmic ointments, injections, suppositories, nasal preparations, and the like. Preferred formulations of the agent/composition of the present invention include eye drops and eye ointments.

Pharmaceutically acceptable additives that may be comprised in the agent/composition of the present invention are not particularly limited and may be selected as appropriate according to the route of administration, formulation, etc. Examples of such pharmaceutically acceptable additives include, for example, surfactants, buffers, tonicity agents, stabilizers, preservatives, antioxidants, thickeners, solubilizing agents, suspending agents, bases, solvents, pH adjusters, excipients, disintegrating agents, binders, fluidizers, lubricants, preservatives, antioxidants, coloring agents, sweetening agents, and the like.

When the agent/composition of the present invention is an eye drop, examples of additives that may be used include surfactants, buffers, tonicity agents, stabilizers, preservatives, antioxidants, thickeners, solvents, pH adjusters, and the like.

Examples of surfactants include cationic surfactants, anionic surfactants, nonionic surfactants and the like.

When a surfactant is added to the agent/composition of the present invention, the amount of the surfactant comprised in the agent may be appropriately adjusted depending on the type of the surfactant, etc., and is preferably, for example, 0.01 to 1% (w/v).

Examples of buffers include phosphoric acid or salts thereof, which may be hydrates or solvates thereof.

Examples of the phosphoric acid or salts thereof include phosphoric acid, trisodium phosphate, sodium dihydrogenphosphate, sodium hydrogen phosphate (disodium hydrogenphosphate) and the like, which may be hydrates thereof.

When a buffer is added to the agent/composition of the present invention, the amount of the buffer comprised in the agent may be appropriately adjusted depending on the type of the buffer, etc., but for example, 0.001 to 10% (w/v) is preferable, and 0.01 to 5% (w/v) is more preferable. Two or more kinds of buffers may be used together.

Examples of tonicity agents include ionic tonicity agents and nonionic tonicity agents. Examples of the ionic tonicity agents include sodium chloride and the like.

When a tonicity agent is added to the agent/composition of the present invention, the amount of the tonicity agent comprised in the agent may be appropriately adjusted according to the type of the tonicity agent or the like, but for example, 0.001 to 10% (w/v) is preferable, and 0.01% to 5% (w/v) is more preferable.

Examples of thickeners include hydroxypropyl methylcellulose and the like.

When a thickener is added to the agent/composition of the present invention, the amount of the thickener may be appropriately adjusted according to the type of the thickener or the like, but for example, 0.001 to 5% (w/v) is preferable, and 0.01% to 3% (w/v) is more preferable.

When the agent/composition of the present invention is an aqueous formulation (e.g., eye drops), the pH is preferably 4 to 8 and more preferably 5 to 7.

Examples of solvents include water, physiological saline and the like.

Examples of the agent/composition of the present invention which is an aqueous preparation (e.g., eye drop) include aqueous preparations comprising the compound of the present invention, water, and an additive selected from ethyl pyruvate, sodium dihydrogenphosphate monohydrate ($NaH_2PO_4H_2O$), disodium hydrogenphosphate ($Na_2HPO_4$), hydroxypropyl methylcellulose, NaCl, polyoxyl 35 castor oil, benzyl benzoate, and a mixture thereof. Here, said "a mixture thereof" means any combination of the listed specific additives.

As used herein, the term "an effective amount" is the amount of the active ingredient required to provide a patient benefit in the symptoms of a disease.

A dosage and administration of the agent/composition of the present invention is not particularly limited as long as the dosage and administration are sufficient to achieve the desired medicinal effect, and may be appropriately selected according to the symptoms of the disease, the age and weight of the patient, the dosage form of the agent, etc.

For example, in the case of eye drops, a single dose of 1 to 5 drops (preferably 1 to 3 drops, more preferably 1 to 2 drops, particularly preferably 1 drop) may be instilled 1 to 4 times per day (preferably 1 to 3 times per day, more preferably 1 to 2 times per day, particularly preferably once per day), every day or at an interval of from one day to one week. The "one drop" is usually about 0.01 to about 0.1 mL, preferably about 0.015 to about 0.07 mL, more preferably about 0.02 to about 0.05 mL, and particularly preferably about 0.03 mL.

In one embodiment, the agent of the present invention have an immediate effect on presbyopia, an eye disease accompanied by a decrease in lens elasticity, or an eye disease accompanied by a decrease in accommodative function of the eye, for example, compared to EV06 or other lipoic acid prodrug(s).

The duration of administration of the agent of the present invention may be determined by a physician or professional.

In one embodiment, the agent of the present invention may be an ophthalmic administration agent such as an eye drop (e.g., solution, emulsion, suspension) and an eye ointment, and may be used continuously for at least 2 days, at least 3 days, at least 7 days, at least 10 days.

In one embodiment, the agent of the present invention may be administered at least once (e.g., at least twice, at least three times) a day.

In one embodiment, the agent of the present invention, when administered to the eye, may be less irritating to the eye while having an effect on presbyopia, an eye disease accompanied by a decrease in lens elasticity, or an eye disease accompanied by a decrease in accommodative function of the eye.

Synthesis

A number of methods well known to those skilled in the art of organic synthesis can be used to prepare The compound of the invention. The compound of the present invention may be synthesized using methods of the following examples, or variations thereof well recognized by those skilled in the art, in conjunction with synthetic methods known in the art of organic synthetic chemistry. Preferred methods include, but are not limited to, those described in the following examples.

For example, the compound of the present invention can be synthesized according to Synthetic route 1. That is, the compound of the present invention is obtained by reacting a commercially available lipoic acid (a-lipoic acid) with an alcohol (I) in the presence of a base such as 4-dimethylaminopyridine, in an organic solvent such as t-butyl methyl ether (hereinafter referred to as "MTBE"), and in the presence of a condensing agent such as diisopropylcarbodiimide (hereinafter referred to as "DIC") at room temperature for 1 to 24 hours. The alcohol (I) used in this synthetic route may be a commercially available compound or a compound prepared from a commercially available compound by a generally used synthetic method.

Synthetic route 1

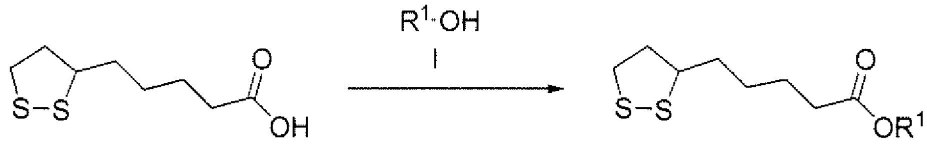


α-Lipoic acid II

Examples

The synthesis of compounds and the results of pharmacological, pharmacokinetic, and safety studies are shown below for a better understanding of the present invention and are not intended to limit the scope of the present invention.

[Synthesis of Compound]

Intermediate 1

2-(4-(2-((tert-Butyldimethylsilyl)oxy)ethyl)piperazin-1-yl)ethan-1-ol (Intermediate 1)

To a solution of 1,4-bis(2-hydroxyethyl)piperazine (CAS RN 122-96-3, 5.00 g, 28.7 mmol) in chloroform (500 mL) were added tert-butyldimethylsilyl chloride (4.33 g, 28.7 mmol) and triethylamine (4.0 mL, 28.7 mmol) at room temperature under a nitrogen atmosphere, and the mixture was stirred overnight. The reaction mixture was concentrated, and the residue was purified twice by silica gel column chromatography (chloroform/methanol = 100/1 to 5/1) to give the title intermediate 1 (1.71 g , 21%) as a colorless oil.

| | |
|---|---|
| 2-(4-(2-((*tert*-Butyldimethylsilyl)oxy)ethyl)piperazin-1-yl)ethan-1-ol (Intermediate 1) 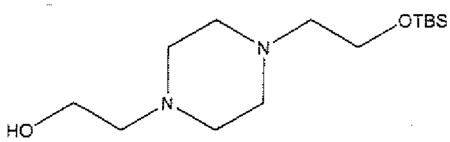 | $^1$H-NMR (400 MHz, $CDCl_3$) δ 0.05 (s, 6H), 0.88 (s, 9H), 2.45 - 2.61 (m, 12H), 3.60 (t, 2H, J=6.0 Hz), 3.76 (t, 2H, J=6.0 Hz) |

Intermediate 2

2-(4-(2-((*tert*-Butyldimethylsilyl)oxy)ethyl)piperazin-1-yl)ethyl (*R*)-5-(1,2-dithiolan-3-yl)pentanoate (Intermediate 2)

To a solution of (R)-α-lipoic acid (1.22 g, 5.91 mmol), 2-(4-(2-((*tert*-butyldimethylsilyl)oxy)ethyl)piperazin-1-yl)ethan-1-ol (Intermediate 1, 1.71 g, 5.93 mmol), and 4-dimethylaminopyridine (217 mg, 1.78 mmol) in MTBE (24 mL) was added dropwise a solution of DIC (821 mg, 6.51 mmol) in MTBE (7.5 mL) at room temperature under a nitrogen atmosphere, and the resulting reaction mixture was stirred at room temperature overnight. The reaction mixture was filtered, the filtrate was concentrated in vacuo, and the residue was purified by silica gel column chromatography (ethyl acetate/methanol = 100/0 to 9/1) to give the title intermediate 2 (2.35 g, 83%) as a yellow oil.

| | |
|---|---|
| 2-(4-(2-((tert-Butyldimethylsilyl) oxy) ethyl)piperazin-1-yl)ethyl (R)-5-(1,2-dithiolan-3-yl)pentanoate (Intermediate 2)<br>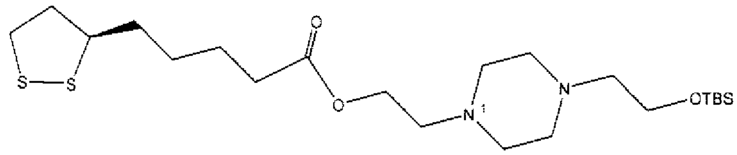 | $^1$H-NMR (400 MHz, $CDCl_3$)<br>δ 0.05 (s, 6H), 0.88 (s, 9H), 1.43-1.50 (m, 2H), 1.63-1.73 (m, 5H), 1.88-1.94 (m, 1H), 2.33 (t, J=6.8 Hz, 2H), 2.42-2.54 (m, 10H), 2.63 (t, J=6.0 Hz, 2H), 3.08-3.20 (m, 2H), 3.55-3.59 (m, 1H), 3.75 (t, J=6.4Hz, 2H), 4.20 (t, J=6.4 Hz, 2H). |

Intermediate 3

1,3-Dimorpholinopropan-2-ol (Intermediate 3)

1,3-Dichloropropan-2-ol (500 μl, 5.31 mmol) and morpholine (3.0 ml) were mixed at room temperature under a nitrogen atmosphere, and the mixture was stirred at 150 °C for 30 minutes using a microwave apparatus. To the reaction mixture was added a 10% aqueous sodium hydroxide solution, and the mixture was extracted with methylene chloride/methanol (10/1). The organic layer was dried over anhydrous sodium sulfate, filtered and concentrated under reduced pressure. The resulting residue was purified by silica gel column chromatography (methylene chloride/methanol = 100/0 to 9/1) to give the title intermediate 3 (872 mg , 71%) as a yellow oil.

| | |
|---|---|
| 1,3-Dimorpholinopropan-2-ol (Intermediate 3)<br>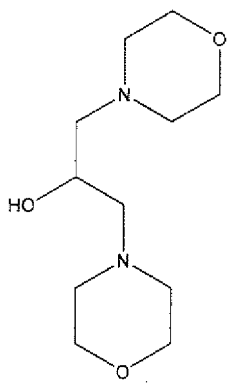 | $^1$H-NMR(400 MHz, $CDCl_3$)<br>δ2.36 (d, J=6.0 Hz, 4H), 2.46-2.50(m, 4H), 2.57-2.62(m, 4H), 2.57-2.62(m, 4H), 3.68-3.76(m, 4H), 3.91(quin, J=6.0 Hz, 1H). |

Compound 1

3-Methyl-2-buten-1-yl (*R*)-5-(1,2-dithiolan-3-yl)pentanoate (Compound 1)

To a solution of (*R*)-α-lipoic acid (3.0 g, 14.6 mmol), 3-methyl-2-buten-1-ol (1.38 g, 16.0 mmol), and 4-dimethylaminopyridine (534 mg, 4.37 mmol) in MTBE (24mL) was added dropwise a solution of DIC (2.02 g, 16.0 mmol) in MTBE (12 mL) at room temperature under a nitrogen atmosphere, and the resulting reaction mixture was stirred at room temperature overnight. The reaction solution was filtered, the filtrate was concentrated in vacuo, and the resulting residue was purified by silica gel column chromatography (hexane/ethyl acetate = 100/0 to 9/1) to give the title compound 1 (1.71 g, 43%) as a yellow oil.

| | |
|---|---|
| 3-Methyl-2-buten-1-yl (R)-5-(1,2-dithiolan-3-yl)pentanoate (Compound 1)<br>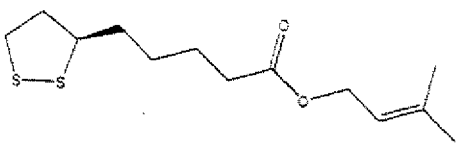 | $^1$H-NMR (400 MHz, DMSO -$d_3$)<br>δ 1.40-1.60 (m, 2H), 1. 60-1.75 (m, 7 H), 1.77( s. 3H), 1.87-1.96 (m. 1 H), 2. 33 (t, J = 8.0 H z. 2H), 2. 43-2. 52 (m, 1 H), 3.09-3.23 (m. 2H), 3. 54- 3. 61 (m, 1H), 4. 5 8 (d, J = 6.8 Hz, 2H), 5. 33-5. 38 (m. 1H). |

Compound 2

(*R*)-2-((5-(1,2-Dithiolan-3-yl)pentanoyl)oxy)-*N*-benzyl-*N,N*-dimethylethan-1-aminium chloride (Compound 2)

Using a commercial available benzyl(2-hydroxyethyl)dimethylammonium chloride and using chloroform instead of MTBE as a solvent, the title compound 2 was obtained in a similar manner to Compound 1 (35% yield).

| | |
|---|---|
| (R)-2-((5-(1,2-Dithiolan-3-yl)pentanoyl)oxy)-N-benzyl-N,N-dimethylethan-1-aminium chloride (Compound 2)<br>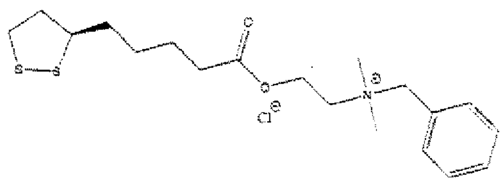 | $^1$H-NMR (400 MHz, $CD_3OD$)<br>$\delta$ 1.43-1.53 (m. 2H), 1.5 8 - 1.75 (m, 4H), 1.84-1. 9 3 (m, 1H), 2. 41 - 2. 50 (m , 3H), 3. 06-3. 20 (m, 8H) 3. 53 - 3.61 (m, 1H), 3.70 - 3.73 (m. 2H), 4.61 (b r s, 4H), 7. 52-7. 61 (m, 5H ). |

Compound 3

2-(4-(2-Hydroxyethyl)piperazin-l-yl)ethyl (R)-5-(1,2-dithiolan-3-yl)pentanoate (Compound 3)

To a solution of Intermediate 2 (2.35 g, 4.93 mmol) in methylene chloride (24 mL) was added tetrabutylammonium fluoride (1 M tetrahydrofuran solution, 14.8 mL, 14.8 mmol) at room temperature under a nitrogen atmosphere, and the resulting reaction mixture was stirred at room temperature for 4 hours. The reaction solution was concentrated in vacuo and the resulting residue was purified by silica gel column chromatography (ethyl acetate to chloroform/methanol=5/1), followed by NH silica gel column chromatography (ethyl acetate/chloroform=9/1 to 1/1 to chloroform/methanol=9/1) to give the title compound 3 (1.24 g , 69%) as a yellow oil.

| | |
|---|---|
| 2-(4-(2-Hydroxyethyl)piperazin-1-yl)ethyl (*R*)-5-(1,2-dithiolan-3-yl)pentanoate (Compound 3)<br>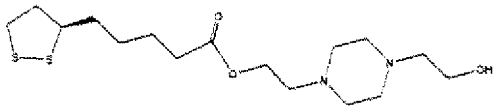 | $^1$H-NMR (400 MHz, CDC $I_3$)<br>$\delta$ 1. 46-1. 70 (m, 7H), 1 . 86-1. 96 (m. 1H), 2. 3 4 (t, J = 6. 8 Hz, 2H) , 2. 42-2. 54 (m. 11H) . 2. 64 (t, J = 6.0 Hz, 2H), 3. 08-3. 22 (m, 2H ), 3. 55- 3. 6 2 (m, 3H), 4. 21 (t, J = 6.0 Hz, 2H). |

Compound 4

2-Morpholinoethyl (*R*)-5-(1,2-dithiolan-3-yl)pentanoate (Compound 4)

Using a commercially available 2-morpholinoethanol, the title compound 4 was obtained in a similar manner to Compound 1 (21% yield).

| | |
|---|---|
| 2-Morpholinoethyl (*R*)-5-(1,2-dithiolan-3-yl) pentanoate (Compound 4)<br>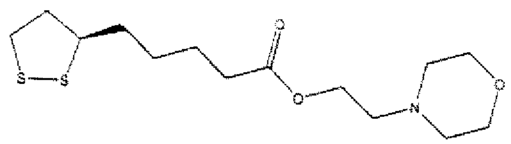 | $^1$H-NMR(400 MHz, $CDCl_3$)<br>$\delta$ 1.44- 1.53 (m, 2H), 1. 62-1. 74 (m, 4H), 1. 88-1.94 (m, 1H), 2.34 (t. J = 7.6 Hz, 2 H), 2. 44 - 2.52 (m, 5H), 2.62 (t. J = 6.0 Hz, 2H), 3.10 - 3.21 (m, 2H), 3.55 - 3.59 (m, 1H), 3. 69-3. 73 (m 4H), 4. 21 (t, J = 6. 0 Hz, 2H). |

Compound 5

1,3-Dimorpholinopropan-2-yl (R)-5-(1,2-dithiolan-3-yl)pentanoate (Compound 5)

To a solution of 1,3-dimorpholinopropan-2-ol (Intermediate 3, 491 mg, 2.13 mmol), $\alpha$-lipoic acid (400 mg, 1.94 mmol), and 4-dimethylaminopyridine (71.1 mg, 0.582 mmol) in MTBE (3.2 ml) was added dropwise a solution of DIC

(330 μl, 2.13 mmol) in MTBE (1.6 ml) at room temperature under a nitrogen atmosphere, and the resulting reaction mixture was stirred at room temperature overnight. The reaction solution was filtered, the filtrate was concentrated in vacuo, and the resulting residue was purified by silica gel column chromatography (hexane/ethyl acetate=9/1 to 0/100 to ethyl acetate/methanol=9/1) to give the title compound 5 (335 mg , 41%) as a yellow oil.

| | |
|---|---|
| 1,3-Dimorpholinopropan-2-yl (*R*)-5-(1,2-dithiolan-3-yl)pentanoate (Compound 5)<br>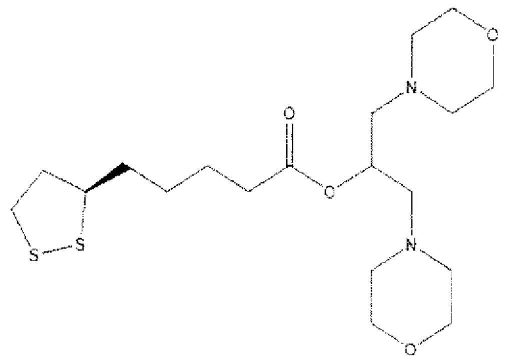 | δ $^1$H-NMR (400 MHz, $CDCl_3$)<br>δ 1. 44-1. 62 (m, 2H) . 1. 6 2-1. 76 (m, 4H), 1. 87-1. 96 (m, 1H), 2. 33 (t, J = 7.6 Hz, 2H), 2. 39-2. 56 (m, 13 H), 3. . 22 (m. 2H), 3. 53- 3. 6 1 (m, 1H), 3. 63-3. 69 (m, 8H), 5. 21 (quin, J = 6. 4 Hz, 1H). |

Compound 6

(2,2,5-Trimethyl-4H-[1,3]dioxo[5,4-c]pyridin-8-yl)methyl (R)-5-(1,2-dithiolan-3-yl)pentanoate (Compound 6)

A slurry solution of (2,2,8-trimethyl-4H-[1,3]dioxo[4, 5-c]pyridin-5-yl)methanol (1.00 g, 4.77 mmol), α-lipoic acid (1.18 g, 5.72 mmol), 4-dimethylaminopyridine (0.21 g, 1.72 mmol) in MTBE (9.4 ml) was prepared at room temperature under argon atmosphere, and DIC (0.98 ml, 4.27 mmol) was added to the slurry under ice cooling. After 5 minutes, the mixture was brought to room temperature and stirred for 3 hours and 40 minutes. The reaction mixture was filtered, and the filtrate was concentrated in vacuo, and the resulting residue was purified by silica gel column chromatography (chloroform/methanol = 40/1) to give the title compound 6 (1.94 g , 85%) as a yellow oil.

| | |
|---|---|
| (2,2,5-Trimethyl-4H-[1,3]dioxo[5,4-clpyridin-8-yl)methyl (*R*)-5-(1,2-dithiolan-3-yl)pentanoate (Compound 6)<br>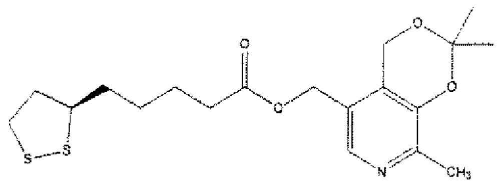 | δ $^1$H-NMR (400 MHz, $CDCl_3$)<br>δ 1.32-1. 53 (m. 2H), 1. 5 6 (s, 6 H), 1.60 - 1. 74 (m, 4H) . 1. 84-1. 93 (m, 1H), 2. 33-2. 36 (m, 2H), 2. 42 (s. 3H) . 2.42-2. 50 (m, 1 H) . 3. 08-3. 22 (m, 2H), 3 . 50-3. 5 7 (m. 1H), 4. 86 ( s, 2H) . 5. 00 (s. 2H), 8. 0 3 (s, 1H). |

[Pharmacological test-1]

The effects of compounds 1 to 4 on the lens elasticity were examined. The tests were conducted with reference to the methods described in Invest Ophthalmol Vis Sci, 57, 2851-2863, 2016.

(Preparation of Test sample)

1) Preparation of Vehicle

A vehicle (aqueous solution) comprising 0.1% (w/v) of ethyl pyruvate, 0.269% (w/v) of sodium dihydrogenphosphate monohydrate ($NaH_2PO_4.H_2O$), 0.433% (w/v) of disodium hydrogenphosphate ($Na_2HPO_4$), 0.2% (w/v) of hydroxypropyl methylcellulose, 0.5% (w/v) of NaCl, 1.0% (w/v) of polyoxyl 35 castor oil (hereinafter also referred to as "CO35"), and purified water (appropriate amount) was prepared.

2) Preparation of Samples of compounds 1 to 4

Each of compounds 1 to 4 was stirred with the addition of the vehicle to give a 0.03% (w/v) solution.

3) Preparation of EV06 sample

EV06 is a compound represented by the following formula:

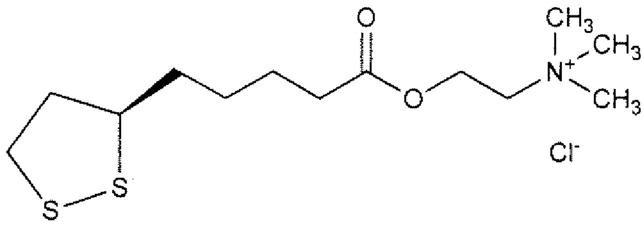

EV06 was stirred with the addition of the vehicle to give a 0.03% (w/v) solution.

(Test method)

1) Each test sample (2.5 μL/eye) was instilled into the right eye of 8-month-old C57BL/6J mice with a Pipetman 3 times per day (around 9:00, 13:00, 17:00) for 12 to 15 days.
2) After the final instillation, the mice were euthanized by carbon dioxide inhalation, and then the eyeballs were extracted and rinsed with Hank's balanced salt solution (HBSS).
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 x 22 mm Square) was placed on the lens, and an image in which the thickness of the lens changed due to the weight was similarly captured (Image b).
6) A change in the lens diameter was calculated from the following Equation 1 wherein the lens diameter of Image a is subtracted from the lens diameter of Image b. Then, the lens elasticity improvement of each sample group compared with the vehicle control group was calculated from Equation 2 described below. The mean value of the vehicle control group was based on 5 eyes, the mean value of each of the sample groups of compounds 1 to 4 was based on 10 eyes, and the mean value of EV06 sample group was based on 9 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each Test sample group - Mean change in lens diameter of Vehicle control group

(Results)

The results are shown in Table 1.

[Table 1]

| Table 1 | Lens elasticity improvement (μm) |
|---|---|
| 0.03% Compound 1 sample | 48.0 |
| 0.03% Compound 2 sample | 6.1 |
| 0.03% Compound 3 sample | 49.9 |
| 0.03% Compound 4 sample | 37.5 |
| 0.03% EV06 sample | 4.0 |

As shown in Table 1, at a concentration of 0.03%, Compounds 1, 3, and 4 sample groups showed a potent lens elasticity improving effect, and Compound 2 and EV06 sample groups did not show lens elasticity improving effect.

[Pharmacological test-2]

The effects of Compounds 1, 3, and 4 on the lens elasticity were investigated at different concentrations.

(Preparation of Test sample)

1) Preparation of Vehicle

Vehicle A

Vehicle A (aqueous solution) comprising 0.1% (w/v) of ethyl pyruvate, 0.269% (w/v) of sodium dihydrogen-phosphate monohydrate ($NaH_2PO_4.H_2O$), 0.433% (w/v) of disodium hydrogenphosphate ($Na_2HPO_4$), 0.2% (w/v) of hydroxypropyl methylcellulose, 0.5% (w/v) of NaCl, 0.2% (w/v) of CO35, and purified water (appropriate amount) was prepared.

Vehicle B

Vehicle B (emulsion) comprising 0.1% (w/v) of ethyl pyruvate, 0.269% (w/v) of sodium dihydrogenphosphate monohydrate ($NaH_2PO_4.H_2O$), 0.433% (w/v) of disodium hydrogenphosphate ($Na_2HPO_4$), 0.2% (w/v) of hydroxypropyl methylcellulose, 0.5% (w/v) of NaCl, 0.2% (w/v) of CO35, 0.6% benzyl benzoate, and purified water (appropriate amount) was prepared.

Vehicle C

Vehicle C (aqueous solution) comprising 0.1% (w/v) of ethyl pyruvate, 0.269% (w/v) of sodium dihydrogen-phosphate monohydrate ($NaH_2PO_4.H_2O$), 0.433% (w/v) of disodium hydrogenphosphate ($Na_2HPO_4$), 0.2% (w/v) of hydroxypropyl methylcellulose, 0.5% (w/v) of NaCl, 1.0% (w/v) of CO35, and purified water (appropriate amount) was prepared.

Vehicle D

Vehicle D (emulsion) comprising 0.1% (w/v) of ethyl pyruvate, 0.269% (w/v) of sodium dihydrogenphosphate monohydrate ($NaH_2PO_4.H_2O$), 0.433% (w/v) of disodium hydrogenphosphate (Na$_2$HPO4), 0.2% (w/v) of hydroxypropyl methylcellulose, 0.5% (w/v) of NaCl, 1.0% (w/v) of CO35, 3.0% benzyl benzoate, and purified water (appropriate amount) was prepared.

2) Preparation of Diluent

A diluent (aqueous solution) comprising 0.1% (w/v) of ethyl pyruvate, 0.269% (w/v) of sodium dihydrogenphos-phate monohydrate ($NaH_2PO_4.H_2O$), 0.433% (w/v) of disodium hydrogenphosphate ($Na_2HPO_4$), 0.2% (w/v) of hydroxypropyl methylcellulose, 0.5% (w/v) of NaCl, and purified water (appropriate amount) was prepared.

3) Preparation of Compound 1 sample

Compound 1 was stirred with the addition of Vehicle C to prepare a 1.5% (w/v) emulsion, and the emulsion was diluted with the diluent to give a 0.3% (w/v) emulsion. Further, the resulting 0.3% (w/v) emulsion was diluted with the diluent to give a 0.03% (w/v) emulsion. Further, the resulting 0.03% (w/v) emulsion was diluted with the diluent to give a 0.003% (w/v) emulsion.

4) Preparation of Compound 3 sample

Compound 3 was stirred with the addition of Vehicle C to give a 1.5% (w/v) solution, and the resulting solution was diluted to give a 0.3% (w/v) solution. Further, the resulting 0.3% (w/v) solution was diluted with Vehicle A to give a 0.03% (w/v) solution. Further, the resulting 0.03% (w/v) solution was diluted with Vehicle A to give a 0.003% (w/v) solution.

5) Preparation of Compound 4 sample

Compound 4 was stirred with the addition of Vehicle D to give a 1.5% (w/v) emulsion, and the resulting emulsion was diluted with the diluent to prepare a 0.3% (w/v) emulsion. Further, the resulting 0.3% (w/v) emulsion was diluted with the diluent to give a 0.03% (w/v) emulsion. Further, the resulting 0.03% (w/v) emulsion was diluted with the diluent to give a 0.003% (w/v) emulsion.

6) Preparation of. EV06 sample

EV06 was stirred with the addition of Vehicle A to give a 1.5% (w/v) solution, which was used for comparison to the samples of Compound 1 and 3.

EV06 was sonicated with the addition of Vehicle B to give a 1.5% (w/v) emulsion, which was used for comparison to Compound 4 sample.

(Test method)

1) Each test sample (2.5 μL/eye) was instilled into the right eye of 7 to 8-month-old C57BL/6J mice with a Pipetman 3 times per day (around 9:00, 13:00 and 17:00) for 12 to 15 days.
2) After the final instillation, the mice were euthanized by carbon dioxide inhalation, and then the eyeballs were extracted and rinsed with Hank's balanced salt solution (HBSS).
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 x 22 mm Square) was placed on the lens, and an image in which the thickness of the lens changed due to the weight was similarly captured (Image b).
6) A change in the lens diameter was calculated from the following Equation 1 wherein the lens diameter of Image a is subtracted from the lens diameter of Image b. Then, the lens elasticity improvement of each sample group compared with the vehicle control group was calculated from Equation 2 described below. The mean value of the vehicle control group was based on 5 eyes, the mean value of each of the sample groups of compounds 1, 3, and 4 was based on 9 to 10 eyes, and the mean value of each EV06 sample group was based on 9 to 10 eyes.

(Equation 1)
Change in lens diameter =
Lens diameter in Image b of each test sample - Lens diameter in Image a of each test sample

```
(Equation 2)
Lens elasticity improvement of each sample group =
Mean change in lens diameter of each Test sample group -
Mean change in lens diameter of Vehicle control group
```

(Results)

The results for Compound 1 are shown in Table 2.

[Table 2]

| Table 2 | Lens elasticity improvement ($\mu$m) |
|---|---|
| 0.003% Compound 1 sample | 13.0 |
| 0.03% Compound 1 sample | 37.1 |
| 0.3% Compound 1 sample | 36.8 |
| 1.5% EV06 sample | 30.8 |

As shown in Table 2, 0.03% and 0.3% Compound 1 sample groups each showed an excellent result equivalent to or better than 1.5% EV06 group, which confirms that Compound 1 has an excellent elasticity improving effect.
The results for Compound 3 are shown in Table 3.

[Table 3]

| Table 3 | Lens elasticity improvement ($\mu$m) |
|---|---|
| 0.003% Compound 3 sample | 1.3 |
| 0.03% Compound 3 sample | 30.7 |
| 0.3% Compound 3 sample | 41.8 |
| 1.5% EV06 sample | 18.2 |

As shown in Table 3, 0.03% and 0.3% Compound 3 sample groups each showed an excellent result equivalent to or better than 1.5% EV06 group, which confirms that Compound 3 has an excellent elasticity improving effect.
The results for Compound 4 are shown in Table 4.

[Table 4]

| Table 4 | Lens elasticity improvement ($\mu$m) |
|---|---|
| 0.003% Compound 4 sample | 25.0 |
| 0.03% Compound 4 sample | 56.5 |
| 0.3% Compound 4 sample | 52.9 |
| 1.5% EV06 sample | 46.8 |

As shown in Table 4, 0.03% and 0.3% Compound 4 sample groups each showed an excellent result equivalent to or better than 1.5% EV06 group, which confirms that Compound 4 has an excellent elasticity improving effect.

[Pharmacological test-3]

The effects of Compounds 5 and 6 on the lens elasticity were investigated.

(Preparation of Test sample)

1) Preparation of Compound 5 sample

Compound 5 was stirred with the addition of Vehicle C to give a 0.03% (w/v) solution.

2) Preparation of Compound 6 sample

Compound 6 was stirred with the addition of Vehicle C to give a 0.03% (w/v) solution.

3) Preparation of EV06 sample

EV06 was stirred with the addition of Vehicle C to give a 0.03% (w/v) solution, which was used for comparison to the samples of Compounds 5 and 6.

(Test method)

1) Each test sample (2.5 μL/eye) was instilled into both eyes of 9-month-old C57BL/6J mice with a Pipetman 3 times per day (around 9:00, 13:00 and 17:00) for 14 days.
2) After the final instillation, the mice were euthanized by carbon dioxide inhalation, and then the eyeballs were extracted and rinsed with Hank's balanced salt solution (HBSS).
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 x 22 mm Square) was placed on the lens, and an image in which the thickness of the lens changed due to the weight was similarly captured (Image b).
6) A change in the lens diameter was calculated from the following Equation 1 wherein the lens diameter of Image a is subtracted from the lens diameter of Image b. Then, the lens elasticity improvement of each sample group compared with the vehicle control group was calculated from Equation 2 described below. The mean value of the vehicle control group was based on 8 eyes, and the mean value of each of the sample groups of Compounds 5 and 6 and EV06 was based on 10 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each Test sample group - Mean change in lens diameter of Vehicle control group

(Results)

The results are shown in Table 5.

[Table 5]

| Table 5 | Lens elasticity improvement (μm) |
|---|---|
| 0.03% Compound 5 sample | 40.7 |
| 0.03% Compound 6 sample | 38.1 |

(continued)

| Table 5 | Lens elasticity improvement (μm) |
|---|---|
| 0.03% EV06 sample | 3.6 |

As shown in Table 5, at a concentration of 0.03%, the sample groups of Compounds 5 and 6 showed a potent lens elasticity improving effect, and EV06 sample group did not show lens elasticity improving effect.

[Pharmacological test-4]

The effect of once-daily instillation of Compound 1 for 2 weeks on the lens elasticity was examined.

(Preparation of Test sample)

1) Preparation of Compound 1 sample

Compound 1 was stirred with the addition of Vehicle A to give a 0.3% (w/v) emulsion, and the emulsion was diluted with the diluent to give a 0.1% (w/v) emulsion. Further, the resulting 0.1% (w/v) emulsion was diluted with the diluent to give a 0.03% (w/v) emulsion. Further, the resulting 0.03% (w/v) emulsion was diluted with the diluent to give a 0.01% (w/v) emulsion.

2) Preparation of EV06 sample

EV06 was sonicated with the addition of Vehicle A to give a 1.5% (w/v) solution, which was used for comparison to Compound 1 sample.

(Test method)

1) Each test sample (2.5 μL/eye) was instilled into the right eye of 8-month-old C57BL/6J mice with a Pipetman once per day (around 9:00) for 14 days.
2) After the final instillation, the mice were euthanized by carbon dioxide inhalation, and then the eyeballs were extracted and rinsed with Hank's balanced salt solution (HBSS) .
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Coming(registered trade mark) 22 x 22 mm Square) was placed on the lens, and an image in which the thickness of the lens changed due to the weight was similarly captured (Image b).
6) A change in the lens diameter was calculated from the following Equation 1 wherein the lens diameter of Image a is subtracted from the lens diameter of Image b. Then, the lens elasticity improvement of each sample group compared with the vehicle control group was calculated from Equation 2 described below. The mean value of the vehicle control group was based on 5 eyes, the mean value of each of the sample groups of compound 1 and EV06 was based on 10 eyes.

(Equation 1)

Change in lens diameter =
Lens diameter in Image b of each test sample - Lens diameter in Image a of each test sample

```
(Equation 2)
Lens elasticity improvement of each sample group =
Mean change in lens diameter of each Test sample group -
Mean change in lens diameter of Vehicle control group
```

(Results)

The results for compound 1 are shown in Table 6.

[Table 6]

| Table 6 | Lens elasticity improvement (μm) |
|---|---|
| 0.01% Compound 1 sample | 12.2 |
| 0.03% Compound 1 sample | 33.8 |
| 0.1% Compound 1 sample | 34.1 |
| 0.3% Compound 1 sample | 39.8 |
| 1.5% EV06 sample | 5.8 |

As shown in Table 6, 0.03%, 0.1% and 0.3% Compound 1 sample groups showed excellent results compared to 1.5% EV06 group even when they were instilled once-daily, which confirms that Compound 1 has an excellent elasticity improving effect.

[Pharmacological test-5]

The effect of once-daily instillation of Compound 1 for 1 week on the lens elasticity was examined.

(Preparation of Test sample)

1) Preparation of Compound 1 sample

Compound 1 was stirred with the addition of Vehicle C to give a 0.1% (w/v) solution. A 0.03% (w/v) solution and a 0.01% (w/v) solution were prepared in the same manner.

(Test method)

1) Each test sample (2.5 μL/eye) was instilled into both eyes of 9-month-old C57BL/6J mice with a Pipetman once per day (around 9:00) for 7 days.
2) After the final instillation, the mice were euthanized by carbon dioxide inhalation, and then the eyeballs were extracted and rinsed with Hank's balanced salt solution (HBSS).
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 x 22 mm Square) was placed on the lens, and an image in which the thickness of the lens changed due to the weight was similarly captured (Image b).
6) A change in the lens diameter was calculated from the following Equation 1 wherein the lens diameter of Image a is subtracted from the lens diameter of Image b. Then, the lens elasticity improvement of each sample group compared with the vehicle control group was calculated from Equation 2 described below. The mean value of the vehicle control group was based on 10 eyes, the mean values of Compound 1 sample group were based on 10 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each Test sample group - Mean change in lens diameter of Vehicle control group

(Results)

The results for Compound 1 are shown in Table 7.

[Table 7]

| Table 7 | Lens elasticity improvement (μm) |
|---|---|
| 0.01% Compound 1 sample | 10.6 |
| 0.03% Compound 1 sample | 20.3 |
| 0.1% Compound 1 sample | 60.1 |

As shown in Table 7, Compound 1 at a concentration of 0.1% showed a potent lens elasticity improvement even when it was instilled once-daily for 1 week.

[Pharmacokinetic study-1]

Single instillations of 1.5% (w/v) eye drops of EV06 and Compounds 1 to 4 into rabbits were performed to evaluate a penetration of lipoic acid into aqueous humor.

(Preparation of eye drop)

Each 1.5% (w/v) eye drop of EV06 and Compounds 1, 3, and 4 was prepared in the same manner as Preparation of Test sample in Pharmacological test-2. The 1.5% (w/v) eye drop of EV06 was prepared using Vehicle C. Further, Compound 2 was stirred with the addition of Vehicle C to give a 1.5% (w/v) eye drop of Compound 2.

(Test method)

A single dose (25 μL) of 1.5% (w/v) EV06 eye drop was applied to one eye of a Japanese white rabbit (male) and a single dose (25 μL) of 1.5% (w/v) eye drop of Compound 1, 2, 3, or 4 was applied to the contralateral eye. At 0.5, 1, 2, and 4 hours after instillation, rabbit eyes were subjected to local anesthesia, and aqueous humor was collected (4 eyes per time point). Lipoic acid concentration in the aqueous humor was measured using a high performance liquid chromatography tandem mass spectrometer (LC-MS/MS).

(Test Results and Discussion)

Table 8 shows the concentrations of lipoic acid in the aqueous humor at 0.5, 1, 2, and 4 hours after instillation of each eye drop and the area under the concentration-time curve (AUC) of lipoic acid in the aqueous humor for 4 hours after instillation.

[Table 8]

| Table 8 | 1.5% EV06 | 1.5% Compound 1 | 1.5% Compound 2 | 1.5% Compound 3 | 1.5% Compound 4 |
|---|---|---|---|---|---|
| Mean concentration of lipoic acid in aqueous humor at 0.5 hours after instillation (ng/mL) | 69.0 | 2608 | 184 | 5088 | 3820 |
| Mean concentration of lipoic acid in aqueous humor at 1 hour after instillation (ng/mL) | 11.5 | 735 | 34.2 | 1310 | 1051 |
| Mean concentration of lipoic acid in aqueous humor at 2 hours after instillation (ng/mL) | 1.20 | 24.8 | 4.90 | 27.6 | 48.1 |
| Mean concentration of lipoic acid in aqueous humor at 4 hours after instillation (ng/mL) | 0.276 | 4.92 | 1.03 | 58.2 | 3.30 |
| AUC for 4 hours after instillation (ng·hr/mL) | 45.2 | 1897 | 126 | 3626 | 2774 |

The concentration and AUC of lipoic acid in the aqueous humor of each of the 1.5% (w/v) eye drops of Compounds 1, 3, and 4 were particularly high compared to the 1.5% (w/v) EV06 group, and these compounds showed a higher intraocular penetration compared to EV06.

[Pharmacokinetic study-2]

Single instillation of 0.03% (w/v) Compound 1 eye drop, 0.03% (w/v) Compound 3 eye drop and 1.5% (w/v) EV06 eye drop into rabbits were each performed to evaluate a penetration of lipoic acid into aqueous humor and lens.

(Preparation of eye drop)

0.03% (w/v) Compound 1 eye drop, 0.03% (w/v) Compound 3 eye drop and 1.5% (w/v) EV06 eye drop were prepared in the same manner as Preparation of Test sample in Pharmacological test-2.

(Test method)

A single dose (25 μL) of 1.5% (w/v) EV06 eye drop was applied to one eye of a Japanese white rabbit (male) and a single dose (25 μL) of 0.03% (w/v) Compound 1 eye drop or 0.03% Compound 3 eye drop was applied to the contralateral eye. At 0.5 and 1 hr after instillation, the rabbit was sacrificed, and then the aqueous humor and lens were extracted. Lipoic acid concentrations in the aqueous humor and lens were measured using a high performance liquid chromatography tandem mass spectrometer (LC-MS/MS).

(Test Results and Discussion)

The concentrations of lipoic acid in the aqueous humor and lens at 0.5 and 1 hr after instillation are shown in Tables 9 and 10, respectively.

[Table 9]

| Lipoic acid concentration in aqueous humor (ng/mL) (Mean ± SD) | Time after instillation (hr) | |
|---|---|---|
| | 0.5 | 1 |
| 0.03%(w/v) Compound 1 eye drop (N = 3 eyes) | 107 ± 52 | 23.8 ± 6.1 |
| 0.03%(w/v) compound 3 eye drop (N = 3 eyes) | 57.8 ± 14.6 | 20.0 ± 12.7 |
| 1.5%(w/v) EV06 eye drop (N = 6 eyes) | 33.4 ± 12.4 | 11.1 ± 10.7 |

[Table 10]

| Lipoic acid concentration in lens (ng/g) (Mean ± SD) | Time after instillation (hr) | |
|---|---|---|
| | 0.5 | 1 |
| 0.03%(w/v) Compound 1 eye drop (N = 3 eyes) | 0.274 ± 0.106 | 0.165 ± 0.032 |
| 0.03%(w/v) compound3 eye drop (N = 3 eyes) | 0.113 ± 0.030 | 0.133 ± 0.106 |
| 1.5%(w/v) EV06 eye drop (N = 6 eves) | 0.118 ± 0.074 | 0.0655 ± 0.0635 |

Lipoic acid concentrations in the aqueous humor and lens after instillation of 0.03% (w/v) eye drops of compounds 1 and 3 were equal to or greater than 1.5% (w/v) eye drop of EV06, which shows high penetration properties of Compounds 1 and 3 into aqueous humor and lens.

[Ocular irritation test-1]

(Preparation of sample)

0.03% (w/v) and 0.3% (w/v) Compound 1 eye drops, and 0.03% (w/v) and 0.3% (w/v) Compound 3 eye drops were prepared in the same manner as Preparation of Test sample in Pharmacological test-2. Vehicle A was used for a vehicle group.

(Test method)

Groups treated with Compound 1 eye drop and Compound 3 eye drop

0.03% (w/v) and 0.3% (w/v) Compound 1 eye drops, 0.03% (w/v) and 0.3% (w/v) Compound 3 eye drops, and the vehicle were each instilled into the left eye of a Japanese White rabbit at a dose of 50 μL/eye with pipette ten times at 30 minute intervals. One hour after the final instillation, ocular irritation of anterior segment of the eye was evaluated according to the modified Draize test, and a slit lamp examination was performed. The contralateral eye was untreated.

The ocular irritation of anterior segment of the eye was scored according to the following criteria:
+0.5: very slight; +1: slight; +2: moderate; +3: severe

(Test result)

The test results are shown in Table 11. No irritation was observed in anterior segment of the eye treated with Compound 1 eye drop. The score of the eye treated with compound 3 was low. No abnormalities were observed in the slit lamp examination of the eyes treated with the eye drops of Compound 1 and Compound 3.

[Table 11]

| Eye drop | Vehicle | 0.03% Compound 1 | 0.3% Compound 1 | 0.03% Compound 3 | 0.3% Compound 3 |
|---|---|---|---|---|---|
| Number of animals | 3 | 3 | 3 | 3 | 3 |

(continued)

| Eye drop | | Vehicle | 0.03% Compound 1 | 0.3% Compound 1 | 0.03% Compound 3 | 0.3% Compound 3 |
|---|---|---|---|---|---|---|
| Irritation Score oi anterior segment of the eye | Flare of palpebral conjunctiva | - | - | - | +1 (1 eye) | +1 (1 eye) |
| | Palpebral conjunctival edema | - | - | - | +0.5 (1 eye) | +0.5 (1 eye) +1 (1 eye) |
| | Bulbar conjunctival hyperemia | - | - | - | +1 (1 eye) | +1 (1 eye) +2 (1 eye) |
| | Hyperemia of nictitating membrane | - | - | - | +1 (1 eye) | +1 (3 eyes) |
| | Discharge | - | - | - | +1 (1 eye) | +1 (3 eyes) |
| Slit lamp observation | | - | - | - | - | - |
| -: No noteworthy findings, Findings and score of the instilled eye (left eye) at 1 hour after the last instillation were described. | | | | | | |

(Discussion)

It is shown that Compound 1 and Compound 3 eye drops are highly safe.

[Ocular irritation test-2]

(Preparation of Sample)

Compound 1 was stirred with the addition of Vehicle C to give a 0.3% (w/v) solution, and the resulting solution was diluted with Vehicle C to give a 0.1% (w/v) solution. Further, the resulting 0.1% (w/v) solution was diluted with Vehicle C to give a 0.03% (w/v) solution.

(Test method)

0.03% (w/v), 0.1% (w/v), and 0.3% (w/v) Compound 1 eye drops were each instilled into the left eye of a Japanese White rabbit at a dose of 50 μL/eye with pipette twice per day at a 6-hour interval for 2 weeks. One hour after the final instillation, ocular irritation of anterior segment of the eye was evaluated using a slit lamp according to the McDonald-Shadduck method, and the lens was observed. At the end of the treatment period, histopathological examination of the eye was performed. The contralateral eye was untreated.

The ocular irritation of anterior segment of the eye was scored according to the following criteria: +1: slight; +2: moderate; +3: severe.

(Test result)

The test results are shown in Table 12. No ocular irritation was observed in eyes instilled with Compound 1 eye drops.

[Table 12]

| Eye drop | 0.03% Compound 1 | 0.1% Compound 1 | 0.3% Compound 1 |
|---|---|---|---|
| Number of animals | 3 | 3 | 3 |

(continued)

| Eye drop | | 0.03% Compound 1 | 0.1% Compound 1 | 0.3% Compound 1 |
|---|---|---|---|---|
| Irritation Score of anterior segment of the eye | Conjunctival hyperemia | | - | |
| | Conjunctival edema | | | |
| | Discharge | - | - | - |
| | Anterior chamber flare | - | - | - |
| | Liqht reflex | - | - | - |
| | Iris | - | - | - |
| | Corneal opacity | | | |
| | Angiogenesis | - | - | - |
| | Corneal epithelial disorder | - | - | - |
| Lens | | - | - | - |
| Histopathological examination of the eye | | | | |
| -: No noteworthy findings, Findings and score of the instilled eye (left eye) at 1 hour after the last instillation were described. | | | | |

(Discussion)

It is shown that the eye drops of Compound 1 are highly safe.

Industrial applicability

The compound/agent/composition of the present invention are useful for treating or preventing eye diseases such as presbyopia etc.

## Claims

1. An agent/composition for treating or preventing presbyopia, comprising a compound of Formula [I]:

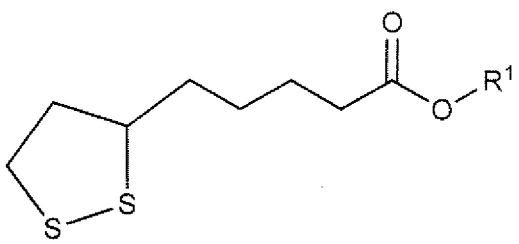

[I]

or a pharmaceutically acceptable salt thereof as an active ingredient, wherein

$R^1$ is selected from:

$C_{2-8}$ alkenyl which may be substituted with the same or different 1 to 4 $R^2$,
$C_{2-8}$ alkynyl which may be substituted with the same or different 1 to 4 $R^2$,
$C_{1-4}$ alkyl substituted with the same or different one or two 4- to 7-membered saturated heteromonocyclic groups, wherein the saturated heteromonocyclic group comprises a carbon atom and the same or different 1 to 3 heteroatoms selected from N, O and $S(O)_{0-2}$ and may be substituted with the same or different 1 to

4 $R^3$, and

$C_{1-4}$ alkyl substituted with the same or different one or two 9- to 10-membered bicyclic fused heterocyclic groups, wherein the bicyclic fused heterocyclic group comprises a carbon atom and the same or different 1 to 4 heteroatoms selected from N, O and $S(O)_{0-2}$ and may be substituted with the same or different 1 to 4 $R^3$;

$R^2$, at each occurrence, is independently selected from -OH, halo, and $C_{1-3}$ alkoxy;
$R^3$, at each occurrence, is independently selected from -OH, halo, $C_{1-3}$ alkyl which may be substituted with the same or different 1 to 4 $R^4$, and $C_{1-3}$ alkoxy which may be substituted with the same or different 1 to 4 $R^4$; and
$R^4$, at each occurrence, is independently selected from -OH and halo.

**2.** An agent/composition for treating or preventing an eye disease accompanied by a decrease in lens elasticity, comprising a compound of Formula [I]:

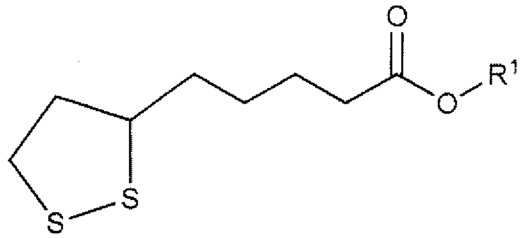

[I]

wherein $R^1$ is as defined in claim 1
or a pharmaceutically acceptable salt thereof as an active ingredient.

**3.** The agent/composition according to claim 2, wherein the eye disease is accompanied by a decrease in accommodative function of the eye.

**4.** An agent/composition for treating or preventing an eye disease accompanied by a decrease in accommodative function of the eye, comprising a compound of Formula [I]:

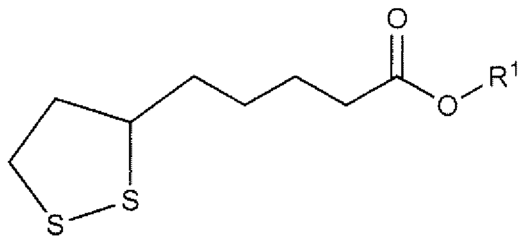

[I]

wherein $R^1$ is as defined in claim 1
or a pharmaceutically acceptable salt thereof as an active ingredient.

**5.** The agent/composition according to any one of claims 2 to 4, wherein the eye disease is presbyopia.

**6.** A composition comprising a compound of Formula [I]:

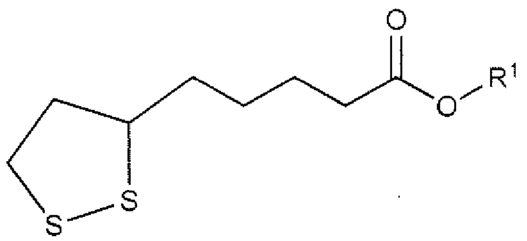

[I]

wherein $R^1$ is as defined in claim 1

or a pharmaceutically acceptable salt thereof,
wherein the composition has a high penetration property into lens.

**7.** The agent/composition according to any one of claims 1 to 6, comprising a compound of Formula [I-a]:

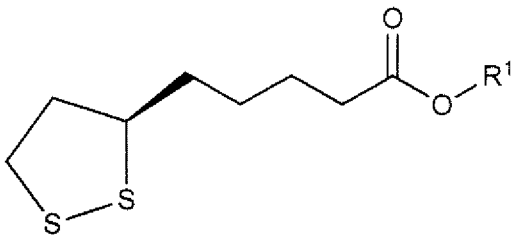

[I-a]

wherein $R^1$ is as defined in claim 1
or a pharmaceutically acceptable salt thereof as an active ingredient.

**8.** The agent/composition according to any one of claims 1 to 7, wherein

$R^1$ is selected from:

$C_{2-8}$ alkenyl which may be substituted with the same or different 1 to 4 $R^2$,
$C_{2-8}$ alkynyl which may be substituted with the same or different 1 to 4 $R^2$, and
$-(CH_2)_{1-4}$-4- to 7-membered saturated heteromonocyclyl, wherein the saturated heteromonocyclyl comprises a carbon atom and the same or different 1 to 3 heteroatoms selected from N, O and $S(O)_{0-2}$ and may be substituted with the same or different 1 to 4 $R^3$;

$R^2$, at each occurrence, is independently selected from -OH, halo, and $C_{1-3}$ alkoxy;
$R^3$, at each occurrence, is independently selected from -OH, halo, $C_{1-3}$ alkyl which may be substituted with the same or different 1 to 4 $R^4$, and $C_{1-3}$ alkoxy which may be substituted with the same or different 1 to 4 $R^4$; and
$R^4$, at each occurrence, is independently selected from -OH and halo.

**9.** The agent/composition according to any one of claims 1 to 8, wherein

$R^1$ is selected from:

$C_{2-8}$ alkenyl which may be substituted with the same or different 1 to 4 $R^2$, and
$C_{2-8}$ alkynyl which may be substituted with the same or different 1 to 4 $R^2$; and

$R^2$, at each occurrence, is independently selected from -OH, halo, and $C_{1-3}$ alkoxy.

**10.** The agent/composition according to any one of claims 1 to 8, wherein

$R^1$ is $C_{1-4}$ alkyl substituted with the same or different one or two 4- to 7-membered saturated heteromonocyclic groups, wherein the saturated heteromonocyclic group comprises a carbon atom and the same or different 1 to 3 heteroatoms selected from N, O and $S(O)_{0-2}$ and may be substituted with the same or different 1 to 4 $R^3$;
$R^3$, at each occurrence, is independently selected from -OH, halo, $C_{1-3}$ alkyl which may be substituted with the same or different 1 to 4 $R^4$, and $C_{1-3}$ alkoxy which may be substituted with the same or different 1 to 4 $R^4$; and
$R^4$, at each occurrence, is independently selected from -OH and halo.

**11.** The agent/composition according to any one of claims 1 to 8, wherein

$R^1$ is $C_{1-4}$ alkyl substituted with the same or different one or two 9- to 10-membered bicyclic fused heterocyclic groups, wherein the bicyclic fused heterocyclic group comprises a carbon atom and the same or different 1 to 4 heteroatoms selected from N, O and $S(O)_{0-2}$ and may be substituted with the same or different 1 to 4 $R^3$;
$R^3$, at each occurrence, is independently selected from -OH, halo, $C_{1-3}$ alkyl which may be substituted with the same or different 1 to 4 $R^4$, and $C_{1-3}$ alkoxy which may be substituted with the same or different 1 to 4 $R^4$; and
$R^4$, at each occurrence, is independently selected from -OH and halo.

**12.** The agent/composition according to any one of claims 1 to 8 and 10, wherein

$R^1$ is $-(CH_2)_{1-4}$-4- to 7-membered saturated heteromonocyclyl, wherein the saturated heteromonocyclyl comprises a carbon atom and the same or different 1 to 3 heteroatoms selected from N, O and $S(O)_{0-2}$ and may be substituted with the same or different 1 to 4 $R^3$;
$R^3$ is selected from -OH, halo, $C_{1-3}$ alkyl which may be substituted with the same or different 1 to 4 $R^4$, and $C_{1-3}$ alkoxy which may be substituted with the same or different 1 to 4 $R^4$; and
$R^4$, at each occurrence, is independently selected from -OH and halo.

**13.** The agent/composition according to any one of claims 1 to 12, comprising as an active ingredient a compound selected from:

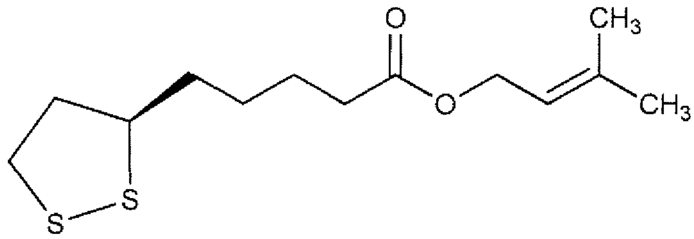

,

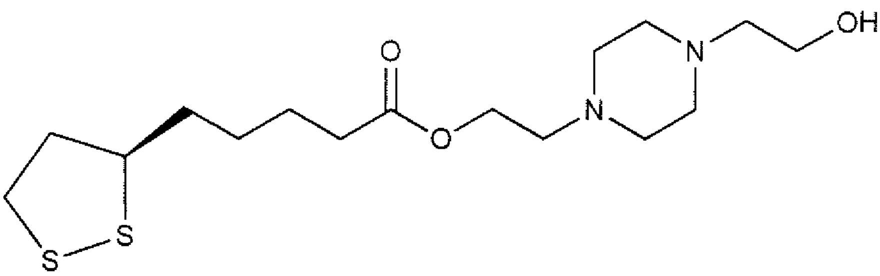

,

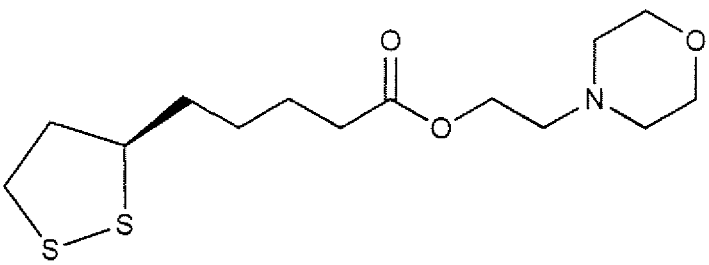

,

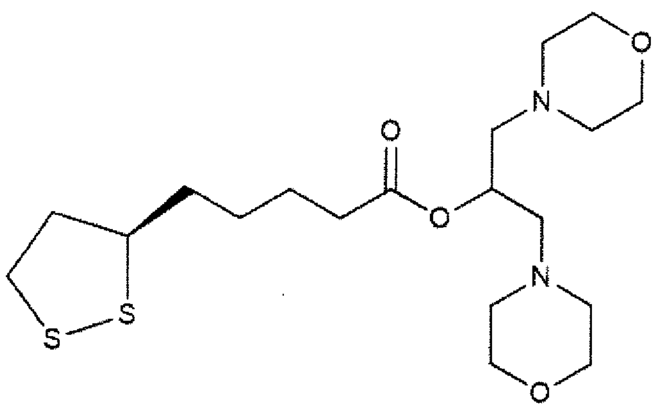

,

and

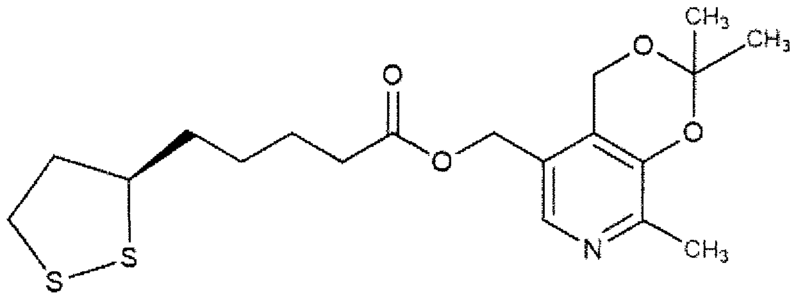

,

or a pharmaceutically acceptable salt thereof.

**14.** The agent/composition according to any one of claims 1 to 13, wherein the agent/composition is for ophthalmic administration.

**15.** The agent/composition according to any one of claims 1 to 14, wherein the agent/composition is an eye drop or an eye ointment.

**16.** The agent/composition according to any one of claims 1 to 15, wherein the amount of the active ingredient comprised in the agent/composition is 0.00001 to 10%(w/v).

**17.** A compound of

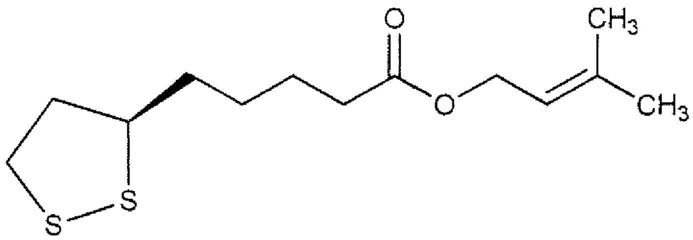

or a pharmaceutically acceptable salt thereof.

## INTERNATIONAL SEARCH REPORT

International application No.
PCT/JP2020/016774

A. CLASSIFICATION OF SUBJECT MATTER
Int. Cl. A61P27/02(2006.01)i, A61P27/10(2006.01)i, C07D339/04(2006.01)i, A61K9/06(2006.01)i, A61K9/08(2006.01)i, A61K31/385(2006.01)i, A61K31/496(2006.01)i, A61K31/5377(2006.01)i
FI: A61K31/385, A61P27/02, A61P27/10, A61K31/496, A61K31/5377, A61K9/08, A61K9/06, C07D339/04 CSP

According to International Patent Classification (IPC) or to both national classification and IPC

B. FIELDS SEARCHED

Minimum documentation searched (classification system followed by classification symbols)
Int. Cl. A61P27/02, A61P27/10, C07D339/04, A61K9/06, A61K9/08, A61K31/385, A61K31/496, A61K31/5377

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2009/111633 A2 (ENCORE HEALTH, LLC) 11 September 2009, claims 1-15, paragraphs [0010], [0011], [0017], [0030] | 1-12, 14-16<br>13, 17 |
| A | JP 2017-511816 A (ENCORE VISION INC.) 27 April 2017, claim 9 | 1-17 |
| A | JP 11-269170 A (SANKYO CO., LTD.) 05 October 1999, paragraphs [0011], [0284], [0285] | 1-17 |

☐ Further documents are listed in the continuation of Box C.

☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08.06.2020 | 16.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/016774

| Patent Documents referred to in the Report | Publication Date | Patent Family | Publication Date |
|---|---|---|---|
| WO 2009/111633 A2 | 11.09.2009 | JP 2008-517911 A<br>JP 2005-506096 A<br>JP 2012-149090 A<br>US 2010/0317608 A1<br>US 2014/0243385 A1<br>WO 2002/013863 A2<br>EP 1808158 A2<br>EP 2939539 A1<br>CA 2584879 A<br>KR 10-2007-0060165 A<br>CN 101083999 A<br>MX 2007004775 A<br>AU 2007202325 A<br>ES 2561600 T | |
| JP 2017-511816 A | 27.04.2017 | US 2016/0354340 A1<br>claim 9<br>WO 2015/134510 A1<br>EP 3113613 A1<br>CA 2941518 A<br>AU 2015227307 A<br>KR 10-2016-0145558 A<br>CN 106455563 A<br>MX 2016011290 A | |
| JP 11-269170 A | 05.10.1999 | US 6013663 A<br>columns 3, second paragraph<br>US 6313164 B1<br>EP 869126 A1<br>EP 1070710 A2<br>CA 2233682 A<br>AU 5970298 A<br>KR 10-1998-0081044 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010147957 A


### Non-patent literature cited in the description


- Atarashii ganka. *A New Ophthalmology,* 2011, vol. 28 (7), 985-988
- *CHEMICAL ABSTRACTS,* 122-96-3
- *Invest Ophthalmol Vis Sci,* 2016, vol. 57, 2851-2863